# FASCICULE DE BREVET EUROPEEN

(11) **EP 0 824 598 B1**
(45) Date de publication et mention de la délivrance du brevet: **06.11.2002**
(21) Numéro de dépôt: 96914259.5
(22) Date de dépôt: 29.04.1996
(51) Int. Cl.: C12Q 1/68, C12N 15/10, C12P 19/34

(54) **METHODE DE COUPLAGE SPECIFIQUE DE LA COIFFE DE L'EXTREMITE 5' D'UN FRAGMENT D'ARNm ET PREPARATION D'ARNm ET D'ADNc COMPLET**
VERFAHREN ZUR SPEZIFISCHEN KOPPLUNG EINER SCHUTZGRUPPE AN DAS 5'-ENDE EINES mRNA-FRAGMENTES UND DIE HERSTELLUNG VON mRNA UND VOLLSTÄNDIGER cDNA
METHOD FOR THE SPECIFIC COUPLING OF THE CAP OF THE EXTREMITY 5' OF A FRAGMENT mRNA AND PREPARATION OF mRNA AND COMPLETE cDNA

(30) Priorité: 02.05.1995 FR 9505221; 03.08.1995 FR 9509467
(43) Date de publication de la demande: 25.02.1998
(73) Titulaire: GENSET SA, 75011 Paris (FR)
(72) Inventeur: MERENKOVA, Irena Nicolaevna, F-75011 Paris (FR); DUMAS MILNE EDWARDS, Jean-Baptiste, Gabriel, F-75006 Paris (FR)
(74) Mandataire: Warcoin, Jacques
(86) Numéro de dépôt international: FR9600651
(87) Numéro de publication internationale: WO96034981

(56) Documents cités:
- EP-A- 0 373 914
- EP-A- 0 373 956
- EP-A- 0 622 457
- EP-A- 0 625 572
- US-A- 4 710 566
- NUCLEIC ACIDS RESEARCH, vol. 14, no. 15, - 1986 pages 6227-45, XP002017390 AGRAWAL, S. ET AL: "Efficient methods for attaching non-radioactive labels to the 5' ends of synthetic oligonucleotides" cité dans la demande
- GENE, vol. 138, 1994, pages 171-174, XP002017391 MARUYAMA, K. ET AL: "oligo-capping: a simple method to replace the cap structure of eurokaryotic mRNAs with oligonucleotides." cité dans la demande

### LISTE DE SEQUENCES

(1) INFORMATIONS GENERALES:
   (i) DEPOSANT:
      (A) NOM: GENSET
      (B) RUE: 1 rue Robert et Sonia Delaunay
      (C) VILLE: Paris
      (E) PAYS: France
      (F) CODE POSTAL: 75011
   (ii) TITRE DE L' INVENTION: METHODE DE COUPLAGE SPECIFIQUE DE LA COIFFE DE L'EXTREMITE 5'D'UN FRAGMENT D'ARNm ET PREPARATION D'ARNm ET D'ADNc COMPLET
   (iii) NOMBRE DE SEQUENCES: 17
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (v) DONNEES DE LA DEMANDE ACTUELLE: NUMERO DE LA DEMANDE: PCT/FR96/00651
(2) INFORMATIONS POUR LA SEQ ID NO: 1:
   (i) CARACTERISTIQUES DE LA SEQUENCE:
      (A) LONGUEUR: 65 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 1:
   CTTAATACGA CTCACTATAG CATCCTACTC CCATCCAATT CCACCCTAAC TCCTCCCATC 60
   TCCAC 65
(2) INFORMATIONS POUR LA SEQ ID NO: 2:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 2:
   CTTAATACGA CTCACTATAG CAT 23
(2) INFORMATIONS POUR LA SEQ ID NO: 3:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 3:
   GTGGAGATGG GAGGAGTTAG GGTG 24
(2) INFORMATIONS POUR LA SEQ ID NO: 4:
   (i) CARACTERISTIQUES DE LA SEQUENCE:
      (A) LONGUEUR: 46 paires de bases
      (B) TYPE: nucléotide
      (C) NOMBRE DE BRINS: simple
      (D) CONFIGURATION: linéaire
   (ii) TYPE DE MOLECULE: ARN (génomique)
   (ix) CARACTERISTIQUE:
      (A) NOM/CLE: misc_feature
      (B) EMPLACEMENT:1
      (D) AUTRES INFORMATIONS:/label= m7Gppp /note= "N représente m7GpppG"
   (xi) DESCRIPTION DE LA SEQUENCE: SEQ ID NO: 4:
   NCAUCCUACU CCCAUCCAAU UCCACCCUAA CUCCUCCCAU CUCCAC 46
(2) INFORMATIONS POUR LA SEQ ID NO: 5:
   (i) CARACTERISTIQUES DE LA SEQUENCE:
      (A) LONGUEUR: 46 paires de bases
      (B) TYPE: nucléotide
      (C) NOMBRE DE BRINS: simple
      (D) CONFIGURATION: linéaire
   (ii) TYPE DE MOLECULE: ARN (génomique)
   (ix) CARACTERISTIQUE:
      (A) NOM/CLE: misc_feature
      (B) EMPLACEMENT:1
      (D) AUTRES INFORMATIONS:/label= ppp /note= "N représente pppG"
   (xi) DESCRIPTION DE LA SEQUENCE: SEQ ID NO: 5:
   NCAUCCUACU CCCAUCCAAU UCCACCCUAA CUCCUCCCAU CUCCAC 46
(2) INFORMATIONS POUR LA SEQ ID NO: 6:
   (i) CARACTERISTIQUES DE LA SEQUENCE:
      (A) LONGUEUR: 208 paires de bases
      (B) TYPE: nucléotide
      (C) NOMBRE DE BRINS: simple
      (D) CONFIGURATION: linéaire
   (ii) TYPE DE MOLECULE: ARN (génomique)
   (ix) CARACTERISTIQUE:
      (A) NOM/CLE: misc_feature
      (B) EMPLACEMENT:1
      (D) AUTRES INFORMATIONS:/label= m7Gppp /note= "N représente m7GpppG"
   (xi) DESCRIPTION DE LA SEQUENCE: SEQ ID NO: 6:
   NCUCCUAUCC CACACUCUCU CACCAUCCUC CACUAUCACC UUUACAUCCA AUCCAAUCCC     60
   AAUUACAUAU ACUCAUCCUA ACUCUACCUC UACCCUUCAU UAACUCCAUU UCCAUUCACC    120
   UUCUCCAUAC UAACUCCUUC AUACUAUUCA UUUCAUCUCA CCCUCUACCU CACACAUCUC    180
   CCACCUUAUC UACCCUAUAC CUCUACUC                                       208
(2) INFORMATIONS POUR LA SEQ ID NO: 7:
   (i) CARACTERISTIQUES DE LA SEQUENCE:
      (A) LONGUEUR: 208 paires de bases
      (B) TYPE: nucléotide
      (C) NOMBRE DE BRINS: simple
      (D) CONFIGURATION: linéaire
   (ii) TYPE DE MOLECULE: ARN (génomique)
   (ix) CARACTERISTIQUE:
      (A) NOM/CLE: misc_feature
      (B) EMPLACEMENT:1
      (D) AUTRES INFORMATIONS:/label= ppp /note= "N représente pppG"
   (xi) DESCRIPTION DE LA SEQUENCE: SEQ ID NO: 7:
   NCUCCUAUCC CACACUCUCU CACCAUCCUC CACUAUCACC UUUACAUCCA AUCCAAUCCC     60
   AAUUACAUAU ACUCAUCCUA ACUCUACCUC UACCCUUCAU UAACUCCAUU UCCAUUCACC    120
   UUCUCCAUAC UAACUCCUUC AUACUAUUCA UUUCAUCUCA CCCUCUACCU CACACAUCUC    180
   CCACCUUAUC UACCCUAUAC CUCUACUC                                       208
(2) INFORMATIONS POUR LA SEQ ID NO: 8:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 8:
   ATCAAGAATT CGCACGAGAC CATTA 25
(2) INFORMATIONS POUR LA SEQ ID NO: 9:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 9:
   TAATGGTCTC GTGCGAATTC TTGAT 25
(2) INFORMATIONS POUR LA SEQ ID NO: 10:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 10:
   CCGACAAGAC CAACGTCAAG GCCGC 25
(2) INFORMATIONS POUR LA SEQ ID NO: 11:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 11:
   TCACCAGCAG GCAGTGGCTT AGGAG 25
(2) INFORMATIONS POUR LA SEQ ID NO: 12:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 12:
   AGTGATTCCT GCTACTTTGG ATGGC 25
(2) INFORMATIONS POUR LA SEQ ID NO: 13:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 13:
   GCTTGGTCTT GTTCTGGAGT TTAGA 25
(2) INFORMATIONS POUR LA SEQ ID NO: 14:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 14:
   TCCAGAATGG GAGACAAGCC AATTT 25
(2) INFORMATIONS POUR LA SEQ ID NO: 15:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 15:
   AGGGAGGAGG AAACAGCGTG AGTCC 25
(2) INFORMATIONS POUR LA SEQ ID NO: 16:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 16:
   ATGGGAAAGG AAAAGACTCA TATCA 25
(2) INFORMATIONS POUR LA SEQ ID NO: 17:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADN (génomique)
   (xi) DESCRIPTION DE LA SEQUENCE: SEQ ID NO: 17:
   AGCAGCAACA ATCAGGACAG CACAG 25

## Description

La présente invention concerne une méthode de couplage spécifique de la coiffe de l'extrémité 5' des ARN messagers (ARNm) eucaryotes. La présente invention concerne également une méthode d'isolement d'ARNm d'eucaryotes comportant la coiffe à l'extrémité 5'. En outre la présente invention concerne une méthode de préparation d'ADNc simple brin (sb) complémentaire de l'extrémité 5' d'ARNm ou d'ADNc double brin (db) correspondant à l'extrémité 5' d'ARN ainsi que d'ADNc complet. La présente invention comporte en outre des trousses de réactifs pour la mise en oeuvre desdites méthodes. La présente invention concerne aussi une méthode de capture de protéine reconnaissant des ARN messagers. Enfin, la présente invention concerne également des fragments d'ARNm couplés spécifiquement par la coiffe 5' à une molécule.

L'activité des gènes est gouvernée par des domaines nucléiques situés en amont du site d'initiation de la transcription. Il est généralement admis que la région d'ADN génomique situé dans les 500 premiers nucléotides en amont du site d'initiation de la transcription constituent le promoteur proximal. Celui-ci contient une grande partie des informations nécessaires à la régulation de la transcription. Certaines séquences facilitant ou inhibant la transcription des gènes se situent bien en amont (côté 5') ou en aval (côté 3') du promoteur proximal. La connaissance des séquences régulant l'activité des gènes est primordiale pour comprendre et à plus long terme modifier les mécanismes d'expression des gènes. La détermination de ces domaines régulateurs est grandement facilitée par l'isolement et la caractérisation des extrémités 5' des messagers. Celles-ci peuvent en effet être utilisées comme point d'ancrage dans le génome humain pour ensuite isoler les promoteurs à l'aide des techniques classiques de la biologie moléculaire ou encore permettre l'identification des gènes et le repérage des promoteurs par analyse informatique (séquences homologues) de séquences d'ADN génomique.

Bien que la séquence des extrémités 5' des ARNm soit d'une grande importance, les techniques classiquement utilisées pour caractériser et isoler des ARNm spécifiques passent par l'isolement des clones d'ADNc correspondants. Or par construction, ces clones sont plus ou moins tronqués dans les parties correspondant aux extrémités 5' des ARNm. Des améliorations certaines ont été récemment apportées dans l'isolement et la caractérisation des extrémités 5'. Frohman et Coll. (1) et Delort et Coll. (2) on décrit l'isolement des extrémités 3' des ADNc correspondant aux extrémités 5' des ARNm au moyen d'adjonction d'un homopolymère à leurs extrémités afin de faciliter le clonage de l'ADNc complet après amplification en chaîne par polymérase ("ACP = PCR" en anglais). Toutefois, les exemples d'application rapportés avec ces méthodes concernaient uniquement des ARN messagers relativement abondants. Delort et Coll. (2) ont montré que la méthode était difficilement applicable à des espèces moléculaires faiblement exprimées. L'inefficacité de la méthode est dûe à l'adjonction de séquences homopolymériques à l'extrémité 3' des ADNc grâce à l'activité de la terminale transférase. En effet, dans ce cas, l'amorce utilisée pour initier la synthèse du deuxième brin (dénomée amorce retour) puis éventuellement pour les ACP subséquentes contient nécessairement une queue homopolymérique à son extrémité 3'. Cette queue homopolymérique, de séquence anticomplémentaire de celle ajoutée à l'extrémité de 3' des ADNc, peut s'hybrider sur des séquences homopolymériques internes contribuant ainsi à la genèse de fragment d'ADN plus court que le fragment attendu. Un deuxième facteur limitant la sensibilité de la méthode est lié à l'amplification de fragments d'ADNc synthétisés par amorçage non spécifique. Ces fragments d'ADNc sont autant de cibles possibles pour l'amorce retour pourvu qu'ils présentent une séquence homopolymérique favorable à l'hybridation.

Pour pallier les inconvénients liés à l'adjonction de queue homopolymérique, Dumas Milne Edwards et Coll. (3) ont ligaturé à l'extrémité 3' des ADNc un oligonucléotide fonctionalisé (étiquette) grâce à l'activité de la ARN ligase du phage T₄. La fonctionalisation de l'oligonucléotide visait à empêcher l'auto-ligature des étiquettes. Pour cela, l'oligonucléotide devait présenter une extrémité 5' P et une extrémité 3' sans fonction hydroxyle. L'étiquetage des ADNc simple brin est supérieur à l'adjonction d'une queue homopolymérique, car une séquence définie est ajoutée à la molécule d'ADNc. Aussi, les difficultés liées à l'utilisation d'une amorce ayant une séquence homopolymérique à son extrémité 3' sont totalement éliminées. Ceci a considérablement amélioré l'isolement d'extrémité 3' d'ADNc. Les deux stratégies décrites précédemment présentent encore la limitation de partir d'ADNc c'est-à-dire, du produit de l'activité de la transcriptase. Or il est connu que l'enzyme peut avoir des difficultés à recopier les brins d'ARN messager, dans certaines régions riches en structures secondaires par exemple.

Pour contourner cette difficulté il a été proposé d'étiquetter directement les ARNm (4 - 7). Cette deuxième méthode repose sur l'ajout à l'extrémité 5' des ARNm d'une -séquence totalement ou partiellement ribonucléotidique permettant à la fois de marquer l'extrémité 5' des ARNm et de fournir une séquence d'ancrage pour les manipulations de clonage. L'extrémité 5' des ARNm eucaryotes est protégée par une structure chimique particulière : la coiffe (méthyl-7-guanosine reliée à l'extrémité 5' des ARNs messagers par une liaison 5'-triphosphate). Dans l'état actuel des connaissances, il n'existe pas de moyens biologiques permettant d'ajouter directement une séquence ribonucléotidique à la coiffe d'ARNm. Aussi, pour isoler les ARNs suivant cette méthode, il est nécessaire d'abord d'éliminer la coiffe des ARNm puis d'ajouter la séquence ribonucléotidique choisie. Cette méthode présente l'avantage de marquer spécifiquement les ARN à leur extrémité 5' à l'aide d'une séquence connue qui peut être utilisée comme cible d'amorces spécifiques utilisables pour synthétiser les deuxièmes brins ADNc et éventuellement pour réaliser des ACP. Toutefois, les manipulations réalisées sur les ARN, en particulier le nombre de réactions enzymatiques à réaliser limitent l'usage que l'on peut faire de cette méthode à des quantités importantes d'ARNm. Cette méthode a l'inconvénient de faire intervenir de nombreuses étapes enzymatiques qui peuvent être limitantes car les enzymes peuvent être contaminées par des ARNases, et entre chaque étape les manipulations permettant de purifier les acides nucléiques sont coûteuses en matériel.

Il a enfin été décrit un procédé de purification d'ARNm (8) dans lequel on utilise les propriétés d'une protéine particulière de reconnaître et se lier à la coiffe de l'extrémité 5' des ARNm après sélection des hétéroduplexes ADNc-ARNm. Cette méthode implique en effet la formation préalable d'hétéroduplexes ARNm-ADNc et ne peut donc s'appliquer qu'à des ARNm de configuration linéaire ou préalablement dénaturés. Toutefois, dans les conditions dénaturantes nécessaires à la linéarisation de la molécule d'ARN pour favoriser la fixation de la coiffe, ladite protéine décrite dans ce procédé se dénature également et perd ses propriétés de reconnaissance et d'affinité. Aucune des méthodes décrites à ce jour ne permet donc l'isolement des ARN messagers complets.

Un but de la présente invention est donc de fournir une méthode permettant d'isoler les ARN messagers complets et notamment leur extrémité 5', qui ne présente pas-les inconvénients précités.

Un autre but de la présente invention est de fournir une méthode de préparation d'extrémité 3' d'ADNc simple brin complémentaire de l'extrémité 5' des ARNm et d'ADNc double brin correspondant à l'extrémité 5' des ARNm et notamment d'ADNc complet.

Pour ce faire, la présente invention fournit tout d'abord une méthode de couplage spécifique de la coiffe de l'extrémité 5' d'un fragment d'ARN, notamment d'ARNm eucaryote par une molécule fonctionnalisée par une fonction amine qui comporte les étapes suivantes :
a) on modifie spécifiquement l'extrémité 3' dudit fragment d'ARNm, de sorte que le dernier nucléotide ne comporte plus de groupe OH en positions 2' et 3' ;
b) on réalise une oxydation spécifique de diol en dialdéhyde sur la fonction 2', 3'-cis diol de la méthyl guanosine de l'extrémité 5' desdits fragments d'ARNm ; et
c) on réalise le couplage de la 2', 3' dialdéhyde obtenue à l'étape b) avec ladite fonction amine de ladite molécule biologique.

On entend ici par "molécule fonctionnalisée par une fonction aminé", une molécule qui peut comporter naturellement une fonction aminé ou une molécule à laquelle on a adjoint une fonction amine. On entend par "fonction amine", le groupe amine tel quel ou une fonction comportant un groupe amine réactif tel que, hydrazide, carbazide, thiocarbazide ou semicarbazide.

L'extrémité 5' des ARN messagers est protégée par une structure particulière : la coiffe. La coiffe est formée d'une guanosine méthylée en position 7 et unie à l'extrémité 5' de la première base de l'ARN par une liaison 5'-5' tri-phosphate. Dans certains cas la guanosine est méthylée en positions 2 et 7. Enfin pour de rares messagers et pour de nombreux petits ARN nucléaires, la guanosine est triméthylée en positions 2, 7, 7 (9). Le sucre (ribose) de ce nucléoside particulier a une fonction 2', 3'-cis diol. Dans une molécule d'ARNm seules les deux extrémités (5' avec la coiffe et 3' avec le dernier sucre 2', 3'-OH) présentent ces diols. C'est pourquoi, selon la présente invention, pour diriger le couplage de la molécule biologique contenant une fonction amine spéficiquement vers l'extrémité 5' on modifie spécifiquement l'extrémité 3' desdites molécules d'ARN. Selon la présente invention il ne subsiste plus alors qu'un seul diol en 5' qui est oxydé en 2', 3' dialdéhyde dans des conditions d'oxydation spécifiques bien connues de l'homme de l'art (10, 11). Le dialdéhyde obtenu peut ensuite réagir avec de nombreux réactifs contenant notamment des molécules comportant une fonction amine. Le couplage de molécules photo-activables (10) et la biotinylation de protéines (12), d'oligonucléotides (11) ont déjà été décrits en faisant réagir un dialdéhyde avec de la biotine-hydrazine. La fixation de protéines à l'extrémité d'oligonucléotides synthétiques hydrazinés a été décrite (13). Cependant, la fixation ne se fait jamais sur la coiffe dans le cas d'acides nucléiques.

A l'étape a) on entend par "modification de l'extrémité 3'" la substitution, transformation ou élimination desdits groupes OH de la dernière base ou l'élimination ou l'adjonction à l'extrémité 3' dudit fragment d'ARNm d'un ou plusieurs nucléotides, de sorte que le dernier nucléotide de l'extrémité 3' ne comporte plus de groupe OH en position 2' et/ou 3' du cycle osidique.

L'objectif de l'étape a) est de supprimer la fonction 2', 3' diol de l'extrémité 3' de sorte que l'oxydation spécifique de fonction diol ne se produise pas à l'extrémité 3', mais seulement à l'extrémité 5'.

L'étape a) est facultative dans la mesure où certains fragments d'ARNm ne comportent pas de diol en position 2' et 3' de la dernière base de leur extrémité 3'.

Dans un mode de réalisation, la modification de l'étape a) se fait par adjonction à l'extrémité 3' dudit fragment d'ARNm d'un nucléotide ou d'un oligonucléotide dont l'extrémité 3' ne comporte pas de fonction 2', 3' diol.

Le nucléotide ou l'oligonucléotide adjoint peut présenter à son extrémité 3', par exemple, au moins un groupe OH en 2' et 3' bloqué par un groupe protecteur.

La liaison entre l'extrémité 3' du fragment d'ARNm et le nucléotide ou l'oligonucléotide peut se faire par une liaison internucléotidique 5'-3' avec l'extrémité 5' du nucléotide ou oligonucléotide selon des méthodes connues de l'homme de l'art.

Dans un mode de réalisation particulier, on réalise une ligature d'un nucléoside diphosphate en 3' et 5' (pNp), notamment par l'adjonction de pCp, en utilisant une enzyme, notamment l'ARN ligase et plus particulièrement l'ARN ligase du phage T4 (14-15).

Lorsque le fragment d'ARNm comporte une extrémité 3' polyA se terminant en 3' par une fonction 2', 3' diol à l'étape a), la modification spécifique de l'extrémité-3' dudit fragment d'ARNm est réalisée par hydrolyse alcaline ménagée suivie d'une étape de séparation des fragments à extrémité 3' polyA générés par l'hydrolyse alcaline.

En effet, l'hydrolyse alcaline se fait spécifiquement à l'extrémité 3'. Elle génère plusieurs fragments dont un premier fragment comportant la coiffe en 5' et les autres ont une extrémité 5' OH, et leur extrémité 3' est du type 3'phosphate, 2'phosphate ou (2', 3') cyclophosphate, sauf un fragment comportant l'extrémité 3' du fragment d'ARNm initial qu'il faut donc éliminer.

Cette élimination peut se faire par tous moyens appropriés connus. Les fragments d'ARNm comportent le plus souvent une extrémité 3' constituée par un fragment polyA. Dans ce cas on peut éliminer ledit deuxième fragment, en particulier par fixation sur des oligonucléotides oligo dT, notamment à l'aide d'une colonne de chromatographie comportant une phase solide sur laquelle des oligo dT sont fixés.

L'hydrolyse alcaline ménagée se fait avantageusement en présence de NaOH 0.1M à 4°C pendant 40 à 60 minutes.

L'oxydation des diol du type α-glycol par l'acide périodique (HIO₄) ou par le tétracétate de plomb provoque spécifiquement la coupure de liaison C-C entre les deux fonctions hydroxyl et la formation de dialdéhydes.

Dans un mode de réalisation particulier à l'étape b), ledit réactif d'oxydation spécifique des diols en dialdéhyde est du périodate, notamment de sodium.

Dans un mode de réalisation à l'étape c) les groupements dialdéhydes formés par oxydation réagissent principalement avec des fonctions amines, dans des conditions de pH acide, notamment un pH variant entre 4 et 6. La liaison est ensuite stabilisée par réduction avec un borohydrure tel que NaBH₄ et préférentiellement un cyanoborohydrure tel que NaBH₃CN, pour stabiliser la fonction hydrazone qui se trouve alors transformée en hydrazide.

De façon appropriée ladite molécule fonctionnalisée est une molécule biologique. Comme molécule biologique on peut citer en particulier les protéines telles que l'avidine ou des anticorps, les vitamines ou des molécules ligands entrant en jeu dans des interactions ligand/récepteur, ou encore des oligonucléotides.

La présente invention fournit également une méthode de marquage spécifique de la coiffe de l'extrémité 5' d'un fragment d'ARNm eucaryote, caractérisée en ce qu'on utilise une méthode de couplage selon la présente invention, dans laquelle ladite molécule est une molécule de marquage.

On entend ici par "molécule de marquage" une molécule pouvant être détectée, directement ou indirectement c'est-à-dire après liaison par interaction ou couplage covalent avec une autre molécule et/ou une phase solide. Par "détection directe" on entend notamment les cas où ladite molécule comporte elle-même un élément détectable tel qu'un atome radioactif, ou ladite molécule est couplée à une enzyme que l'on peut détecter à l'aide d'un substrat chromogénique ou ladite molécule est couplée à une molécule fluorescente. Par "détection indirecte" on entend notamment les cas où ladite molécule est susceptible de réagir de manière physico-chimique ou par couplage covalent avec une autre molécule elle-même comportant un élément détectable directement tel qu'un atome radioactif, une enzyme ou une molécule fluorescente.

La présente invention a également pour objet une méthode d'isolement de l'extrémité 5' d'un ARNm eucaryote dans un échantillon biologique, caractérisée en ce qu'on utilise une méthode de couplage selon la présente invention, dans laquelle ladite molécule est une molécule permettant l'isolement du fragment d'ARNm auquel elle est couplée.

On entend par "extrémité 5' d'un fragment d'ARNm" tout fragment qui comprend la coiffe méthyl-guanosine en 5'.

Par "molécule permettant l'isolement", on entend une molécule permettant l'isolement de façon directe ou indirecte du fragment d'ARNm auquel elle est couplée. Par "isolement direct" on peut citer le cas de la précipitation dans le cas d'une protéine. Par "isolement indirect" on peut citer le cas où ladite molécule est susceptible de réagir avec une phase solide ou une deuxième molécule fixée sur une phase solide.

La présente invention a également pour objet une trousse de réactifs utile pour la mise en oeuvre d'une méthode de couplage, de marquage ou d'isolement selon la présente invention, caractérisé en ce qu'elle comporte :
- des réactifs de modification à l'extrémité 3' desdits ARNm ;
- des réactifs d'oxydation de la fonction 2'3'-diol à l'extrémité 5' en dialdéhyde ; et
- des réactifs de couplage covalent entre la dialdéhyde et ladite fonction amine de ladite molécule.

En particulier, ledit réactif d'oxydation est du périodate de sodium.

Selon un mode de réalisation, lesdits réactifs de couplage avec ladite fonction amine comportent un tampon acide à pH entre 4 et 6 et un réactif de réduction consistant en un borohydrure.

Selon une variante avantageuse, lesdits réactifs de modification de ladite extrémité 3' sont des réactifs permettant le couplage de l'extrémité 3' dudit ARNm avec l'extrémité 5' d'un oligonucléotide ou d'un nucléotide ne comportant pas de fonction 2', 3' diol à son extrémité 3'.

En particulier, lesdits réactifs de modification de l'extrémité 3' dudit ARNm consistent en un nucléotide 3', 5' diphosphate pNp et en un enzyme de ligature ARN ligase notamment, l'ARN ligase T4.

La présente invention, permet aussi d'isoler l'extrémité 5' d'ARNm et notamment d'ARNm complets en tirant partie de la réactivité chimique de leur extrémité 5' et de la possibilité de la modifier chimiquement spécifiquement pour l'extrémité 5', de lui fixer une molécule, notamment molécule biologique telle que la biotine. Par la suite, les ARN ainsi marqués peuvent être sélectionnés par les techniques classiques permettant de les sélectioner, par exemple par l'utilisation de billes magnétiques couplées à de la streptavidine dans le cas d'ARN biotinylés. Dans ce cas, l'invention permet de purifier par capture sur bille d'avidine ou de streptavidine des messagers coiffés donc complets ou les extrémités 5' de messagers complets.

Plus précisément, la présente invention a donc en outre pour objet une méthode d'isolement d'ARNm complet notamment à l'extrémité 5', dans un échantillon biologique, caractérisée en ce que :
1) on réalise le marquage spécifique de la coiffe à l'extrémité 5' de l' ARNm avec une première molécule P1 selon la méthode de couplage de la présente invention pour obtenir le conjugué P1-ARNm ;
2) on met ledit ARNm marqué par ladite première molécule biologique (P1-ARNm) en présence d'une deuxième molécule P2 qui interagit et se lie de façon covalente ou non covalente avec ladite première molécule de manière à former un conjugué ou respectivement un complexe (P2 / P1-ARNm) ;
3) on sépare ledit conjugué ou dit complexe P2 / P1-ARNm de l'échantillon et,
4) on effectue éventuellement la coupure de la liaison covalente ou la décomplexation pour récupérer l'ARNm marqué P1-ARNm.

De façon appropriée, à l'étape 2) ladite deuxième molécule se trouve fixée à une phase solide et, à l'étape 3) on sépare la phase solide revêtue dudit conjugué ou complexe de l'échantillon.

Toutefois, si lesdites molécules P1 et P2 sont des protéines, le complexe P2/P1 peut être récupéré par exemple par précipitation, dans ce cas l'usage d'une phase solide n'est pas nécessaire.

Dans un mode de réalisation, comme première (P1) et deuxième molécule (P2), on utilise des molécules biologiques qui interagissent entre elles par une liaison de type non covalent. De tels couples sont bien connus. On peut citer notamment les couples antigène/anticorps tel que digoxigénine (DIG) / anticorps anti-digoxigénine (anti DIG) ou des interéactions entre molécules biologiques de type biotine/avidine ou streptavidine ou encore des réactions d'hybridation entre acides nucléiques complémentaires.

La fixation de P2 sur la phase solide peut être covalente ou non covalente selon des méthodes connues de l'homme de l'art.

Dans un mode de réalisation particulier, P1 est l'hydrazide de biotine et P2 est l'avidine ou la streptavidine. Dans ce cas, on peut séparer l'avidine ou la streptavidine du conjugué biotine-ARNm par simple chauffage, à environ 95°C dans du 2% SDS.

La phase solide peut être constituée par la face interne du récipient dans lequel se trouve l'échantillon biologique ou un élément tel que des billes que l'on introduit dans ledit récipient.

La méthode d'isolement d'ARNm selon l'invention peut comporter une étape supplémentaire de coupure entre P1 et ledit ARNm dans laquelle on sépare ladite première molécule biologique P1, pour récupérer l'ARNm non couplé.

La présente invention a également pour objet une trousse de réactifs d'isolement utile pour la mise en oeuvre d'une méthode d'isolement d'extrémité 5' d'ARNm selon l'invention, caractérisé en ce qu'elle comporte les éléments d'une trousse de réactifs de couplage d'ARNm spécifique de la coiffe en 5' avec une dite première molécule biologique P1 selon la présente invention et une solution de ladite deuxième molécule ou une phase solide sur laquelle se trouve fixée de façon covalente ou non covalente une dite deuxième molécule biologique P2.

La présente invention a également pour objet une méthode de préparation d'extrémité 3' d'ADNc simple brin et notamment d'ADNc complet caractérisée en ce qu'elle comprend les étapes suivantes :
a) on effectue une transcription inverse d'extrémité 5' d'ARNm et notamment d'ARNm complet obtenu par une méthode d'isolement d'extrémité 5' d'ARNm selon la présente invention ;
b) on élimine les ARN simples brins et fragments d'ARN non hybridés pour obtenir une population d'hétéroduplexes ADNc/ARNm dans lesquels l'ARNm est un ARNm complet dont la coiffe en 5' est couplée spécifiquement par une dite première molécule.
c) on capture lesdits hétéroduplexes à l'aide d'une phase solide revêtue de ladite deuxième molécule biologique et,
d) on effectue une déshybridation des hétéroduplexes pour récupérer les fragments d'ADNc simple brin comportant l'extrémité 3' et notamment les ADNc simple brin complets.

Avantageusement à l'étape b), l'élimination des ARN simples brins ou fragments d'ARN non hybridés s'effectue par traitement enzymatique à l'aide d'une enzyme qui dégrade les ARN simples brins ou non hybridés et conserve intacts les hétéroduplexes ARN/ADN.

En particulier, après synthèse d'un ADNc, l'élimination par la RNAse T1 ou des Nucléases S1 des molécules d'ARN ou des fragments d'ARN libres conduit à l'obtention d'une population d'hétéroduplexes contenant une molécule d'ARNm coiffé. Le couplage, notamment la biotinylation de la molécule d'ARN permet sa capture par les méthodes mentionnées ci-dessus et donc la purification de molécules d'extrémité 3' d'ADNc et notamment d'ADNc complets.

La présente invention permet également de réaliser par voie chimique la ligature d'une "étiquette" à la coiffe des ARNs messagers eucaryotes en vue de la préparation d'ADNc double brin, ladite séquence étiquette servant de cibles d'amorces utilisées pour synthétiser les deuxièmes brins d'ADNc. On entend ici par "étiquette", une séquence d'acide nucléique déterminée (désoxyribo, ribonucléotidique ou assimilée) quel que soit le procédé ayant permis d'obtenir cette séquence. Ceci recouvre donc les séquences naturelles ou synthétiques quelle que soit la chimie utilisée pour les obtenir.

La méthode d'étiquetage selon l'invention permet de s'affranchir des difficultés liées à l'utilisation d'enzymes pour réaliser l'étiquetage des extrémités 5' des ARNm. Dans la voie enzymatique d'étiquetage des ARNm impliquant l'élimination préalable de la coiffe, la réaction de ligature est catalysée par une enzyme (la T4 ARN ligase) qui unit une extrémité 3'-OH à une extrémité 5'-P. De ce fait, avant le "décoiffage" des ARNm, il faut inactiver les extrémités 5'-P des ARNs non coiffés, ce qui rajoute une étape enzymatique (catalysée par une phosphatase telle qu'une phosphatase alcaline de bactérie (BAP) ou phosphatase alcaline d'intestin de veau (CIP)). Comme la méthode utilisée se base sur la réactivité de la coiffe, dans une population complexe d'ARN, seuls les ARN messagers coiffés sont étiquetés.

La présente invention a donc également pour objet une méthode de préparation d'ADNc double brin eucaryote correspondant à l'extrémité 5' d'ARNm et notamment d'ADNc complet dans laquelle on effectue les étapes suivantes :
a) On effectue le couplage de la coiffe de l'extrémité 5' d'un fragment d'ARNm eucaryote, selon une méthode de la présente invention dans laquelle ladite molécule fonctionnalisée est un oligonucléotide étiquette comportant une fonction amine à son extrémité 3', et dans laquelle méthode, la première étape de modification de l'extrémité 3' de l'ARNm est facultative,
b) On effectue la transcription inverse de l'ARNm ligaturé obtenu à l'étape a),
c) On dénature les hétéroduplexes obtenus à l'étape b) et on élimine les ARN simples brins pour obtenir le premier d'ADNc, puis
d) On effectue la synthèse du deuxième à l'aide de l'ADN polymérase et d'une amorce dont la séquence est anti-complémentaire de la totalité ou d'une partie de la séquence de l'oligonucléotide étiquette.

On a en effet, découvert selon la présente invention, la possibilité d'effectuer une transcription inverse à travers la coiffe. Cette faculté n'avait jamais été démontrée avant la présente invention.

Cette méthode permet d'obtenir l'ADNc double brin comportant l'extrémité correspondant à l'extrémité 5' de l'ARNm et notamment d'ADNc complet correspondant à l'ARN messager complet.

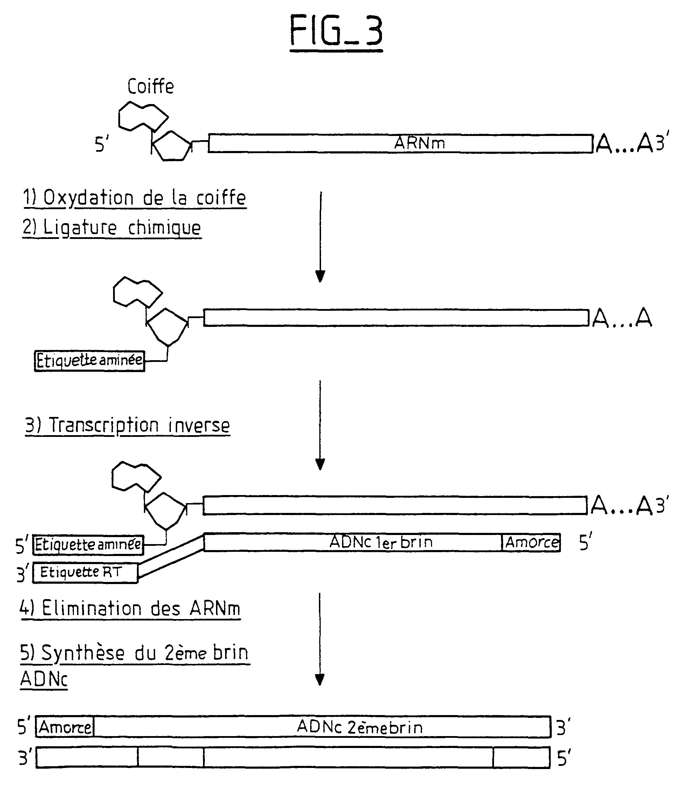

La méthode de ligature chimique de l'étape a) utilise la réactivité des diols, dans une molécule d'ARN coiffée, les deux extrémités du fragment d'ARNm peuvent donc réagir. Une réaction au niveau de la seule coiffe est possible, il faut pour cela bloquer l'extrémité 3' des ARNs coiffés en éliminant la fonction 3'-OH. Toutefois, la ligature d'une étiquette à l'extrémité 3' des ARNm ne limite en rien l'utilisation de l'invention comme cela ressort du schéma de la figure 3. En effet, il s'agit dans ce cas d'une ligature 3'-3' et, de toute façon, les amorces utilisées pour amorcer la transcription inverse sont alors situées en amont de la séquence nucléotidique ajoutée. Pour un meilleur rendement, l'étape de modification de l'extrémité 3' de l'ARNm en vue d'une ligature spécifique de l'étiquette à l'extrémité 5' dudit ARN peut être préférée.

Au cours de l'étape a) du procédé de préparation d'ADNc double brin selon l'invention, on utilise préférentiellement pour la ligation un oligonucléotide étiquette synthétisé par voie chimique. La synthèse de cet oligonucléotide étiquette est réalisée en tenant compte du fait qu'il est essentiel d'utiliser un oligonucléotide non complémentaire du produit d'extension d'amorce, pour éviter l'amplification d'ADN allochtone.

La longueur de l'oligonucléotide étiquette utilisé dans l'invention doit être suffisante pour permettre l'hybridation d'une amorce utilisable par l'ADN polymérase. Préférentiellement, on utilise un oligonucléotide d'une longueur au moins égale à 10 nucléotides. Pour une meilleure mise en oeuvre de l'invention, il peut être préférable d'utiliser un oligonucléotide étiquette ayant une longueur suffisante pour permettre la réalisation d'étapes supplémentaires d'amplification. Dans ces conditions, la longueur de l'oligonucléotide étiquette est avantageusement comprise entre 10 et 40 nucléotides. Dans un mode de réalisation la fonction amine de l'oligonucléotide étiquette est un groupe hydrazide à son extrémité 3'.

Un autre objet de l'invention réside dans une trousse pour la mise en oeuvre du procédé de préparation d'ADNc double brin décrit ci-avant, comprenant notamment l'oligonucléotide étiquette comportant éventuellement une fonction amine à son extrémité 3' et les réactifs permettant sa ligation à l'extrémité 5' de l'ARNm.

Selon une variante particulière de l'invention, la trousse comprend également les amorces et enzymes permettant la transcription inverse de la matrice initiale d'ARN en ADNc-sb et les amorces et enzymes permettant la synthèse du deuxième brin. Selon une autre variante particulière de l'invention, la trousse comprend également les amorces et enzymes permettant l'amplification de l'acide nucléique double brin cloné.

La présente invention a également pour objet une méthode de capture d'une protéine Po reconnaissant un ARNm dans laquelle on utilise de l'ARNm obtenu par une méthode d'isolement selon la présente invention, que l'on met en présence de ladite protéine, puis on récupère le complexe (Protéine/ARNm complet) et on effectue une décomplexation pour récupérer ladite protéine.

Ledit ARNm utilisé peut être couplé à ladite molécule P1 ou en être séparé. De même, ledit ARNm ou conjugué P1-ARNm peut être fixé à une phase solide. En particulier, le conjugué P1-ARNm peut être fixé à une phase solide par l'intermédiaire d'une interaction avec ladite molécule P2 elle-même fixée sur ladite phase solide.

Cette méthode de capture peut s'appliquer en particulier à au moins deux situations biologiquement intéressantes :
- isolement de protéines impliquées dans la régulation de la traduction et de la stabilité des ARN messagers ,
- si les ARNm sont des ARNpré-messagers, la préparation peut être utilisée pour capturer des protéines ou des complexes (SNURPS) intervenant dans l'épissage.

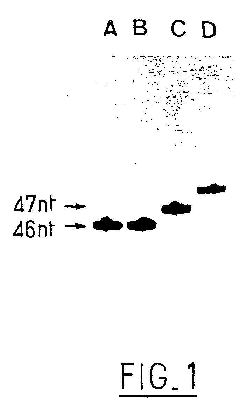

D'autres avantages et caractéristiques de la présente invention apparaîtront à la lumière des exemples de réalisation détaillée qui vont suivre. Ces exemples sont illustrés par la figure 1 qui représente l'électrophorèse sur gel de polyacrylamide, d'oligonucléotides non coiffés (colonnes A et B) et coiffés (colonnes C et D) marqués par ligature de pCp radioactif ³²P et qui ont été soumis au procédé biotinylation de la coiffe décrit dans l'exemple 1 (colonnes B et D) ou non soumis à ce procédé (colonnes A et C).

La figure 2 montre les résultats obtenus lors de la récupération des oligonucléotides biotinylés et capturés par des billes de streptavidine magnétique.

La figure 3 représente le schéma de la ligation chimique d'acides nucléiques à l'extrémité 5' des ARNm. Après purification des ARNm par chromatographie oligo-dT, les ARNs, dont l'extrémité 3'-OH a éventuellement été bloquée, sont oxydés en présence de périodate (étape 1). Puis, une étiquette nucléotidique hydrazinée est ligaturée aux ARNs oxydés (étape 2). La transcription inverse (étape 3) est amorcée (amorce) au moyen d'oligo-dT, d'héxameres à séquence aléatoire ou encore d'amorces ayant une séquence spécifique. Lorsque la transcriptase inverse traverse la coiffe, elle peut recopier l'étiquette (Etiquette RT). Après élimination des ARNs (étape 4), la synthèse des ADNc deuxième brin (étape 5) initié avec une amorce choisie pour s'hybrider avec la séquence de l'étiquette rétro-transcrite (Etiquette RT), conduit à l'obtention d'ADNc double brin contenant au moins l'extrémité 5' des ARNm.

La figure 4 représente une autoradiographie d'un électrophoregramme de gel de polyacrylamide 8% d'oligoribonucléotides de 200 nucléotides marqués au pCp coiffés (colonnes C et D) ou non (colonnes A et B) ligaturés (colonnes B et D) ou non (colonnes A et C) à une étiquette.

La figure 5 représente une autoradiographie d'un électrophoregramme de gel de polyacrylamide 12 % d'oligoribonucléotides de 46 nucléotides marqués au pCp coiffés (colonnes C, D et E) ou non (colonnes A et B) ligaturés (colonnes B, D et E) ou non (A et C) à une étiquette.

La figure 6 représente l'autoradiographie d'un électrophoregramme sur gel de polyacrylamide 12 % des ARNs étiquettés (C,D) ou non (A,B) avant (A,C) ou après (B,D) transcription inverse.

La figure 7 représente l'autoradiogramme d'un gel de polyacrilamide 8% d'oligoribonucléotide de 200nt radiomarqués en 3' par ligature avec du ³²pCp. Colonne A : oligoribonucléotide de 200nt non coiffé, oxydé et ligaturé à l'étiquette hydrazinée. Colonnes B : oligoribonucléotide de 200nt coiffé, oxydé et ligaturé à l'étiquette hydrazinée.

La figure 8 représente une autoradiographie d'une membrane de nylon sur laquelle les ARN étiquetés et des témoins ont été déposés après hybridation d'une sonde radiomarquée (par kination : réaction de phosphorylation) correspondant à un oligodésoxyribonucléotide de séquence anticomplémentaire à celle de l'étiquette. Tous les points sont dupliqués. Colonnes A à D : 0,5, 5, 25 et 50 fmôles d'étiquette. Colonne E : 1/10ème des ARNm étiquetés ont été déposés en deux dépôts.

La figure 9 montre une autoradiographie d'une membrane de nylon sur laquelle les ADNc simples et des témoins ont été déposés après hybridation avec une sonde oligodesoxyribonucléotidique radiomarquée par phosphorylation de son extrémité 5' dont la séquence est identique à celle de l'étiquette. Les colonnes A à E représentent différentes concentrations (1 pmôle, 100 fmôles, 50 fmôles, 10 fmôles et 1fmôle) d'un oligodesoxyribonucléotide témoin de séquence identique à celle de l'étiquette ligaturée. Colonne F : dépôt des ADNc-simple brin étiquetés.

La figure 10 représente la photographie d'un gel d'agarose 1,5% coloré au bromure d'éthidium des produits d'ACP de la transcription inverse (1/20ème des produits de transcription inverse ont été utilisés pour chaque réaction d'ACP).

Colonnes A et B : marqueurs de poids moléculaires (décrire). Colonnes C et D : ACP avec les amorces globines en présence d'ADNc (Colonne C) ou non (Colonne D). Colonnes E et F : ACP avec les amorces deshydrogenase en présence d'ADNc (colonne E) ou non (colonne F). Colonnes G et H : ACP avec les amorces pp15 en présence (colonne G) ou non (colonne H) d'ADNc. Colonnes I et J : ACP avec les amorces EIE4 en présence (colonne I) ou non (colonne J) d'ADNc.

Dans les colonnes C, E, G et I, la présence d'une bande à la taille attendue témoigne de la présence de la séquence correspondante dans la population d'ADNc.

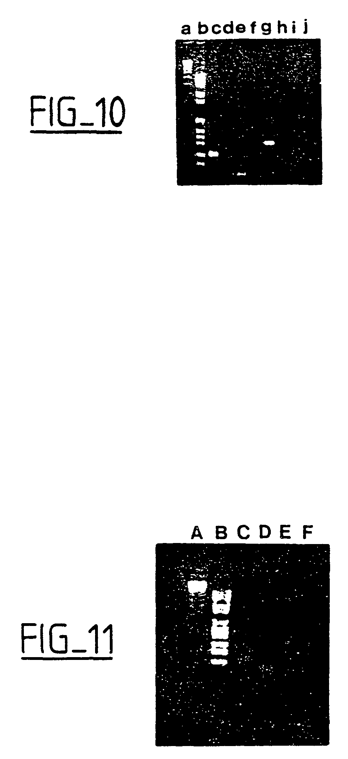

La figure 11 représente la photographie d'un gel d'agarose 1,5% coloré au bromure d'éthidium. Mêmes légendes que la figure 10 mais, les ACP ont été réalisées avec les oligonucléotides anti-sens de chaque couple d'amorce et un oligonucléotide dont la séquence correspond à celle de l'étiquette ligaturée. Les produits d'amplification des colonnes C et E montrent que la transcription inverse a recopié la molécule d'ARN et l'étiquette oligodésoxyribonucléotidique ligaturée.

### EXEMPLE 1 : Marquage spécifique de la coiffe 5' d'ARNm

### 1. Ligature du nucléoside biphosphate pCp à l'extrémité 3' d'ARN messager.

1 µg ARN est incubé dans un milieu réactionnel final de 10 µl en présence de :
a) 5u d'ARN ligase du phage T₄ dans le tampon fournit par le fabricant (Gribco - BRL) et,
b) 40u de l'inhibiteur des ARNase : RNAzine commercialisé par la Société Proméga et,
) 2µl de ³²pCp Amersham #PB 10208. L'incubation peut être réalisée à 37°C pendant 2 heures ou toute la nuit à 7-8°C.

### 2. Obtention de l'ARN sous sa forme coiffée et non coiffée.

L'ARN est obtenu par transcription in vitro à partir d'une matrice d'ADN double brin portant le promoteur de l'ARN polymérase du phase T7.

### 2.1. Production de la matrice d'ADN double brin.

Les matrices d'ADN double brin sont obtenues par PCR de la façon suivante : un oligonucléotide de séquences 5' CT *TAA TAC GAC TCA CTA TAG* CAT CCT ACT CCC ATC CAA TTC CAC CCT AAC TCC TCC CAT CTC CAC 3' (la séquence promotrice de l'ARN polymérase du phage T7 est en italique) (seq.id n° 1) est amplifié en utilisant un primer 5' de séquence CT TAA TAC GAC TCA CTA TAG CAT C 3' (seq. id n°2) et un primer 3' de séquence 5' GTG GAG ATG GGA GGA GTT AGG GTG 3' (seq. id n° 3). Chaque réaction est réalisée dans un volume de 100 µl et contient 2mM MgC12 . 200 µM de chacun des dNTP ; 60 pmoles de chacun des primers ; 1 ng de matrice ADN simple brin et 2,5 unités de Taq ADN polymérase. Les conditions d'amplification sont les suivantes : 1 cycle comprenant 3 min. à 94°C, 30 sec. à 53°C et 30 sec. à 72°C ; 25 cycles comprenant 1 min. à 94°C, 30 sec. à 53°C et 30 sec. à 72°C ; 1 cycle avec 1 min. à 94°C, 30 sec. à 53°C et 5 min. à 72°C. Les produits d'amplification sont alors séparés par électrophorèse sur gel acrylamide 10 % non dénaturant puis visualisés par UV shadowing. Les matrices d'ADN double brin sont alors éluées du gel, soumises à une extraction phénolique puis récupérées par précipitation alcoolique.

### 2.2. Synthèse de l'ARN coiffé ou non coiffé

L'ARN est obtenu par transcription in vitro de la matrice ADN double brin en utilisant le kit de transcription "AmpliScribe T7" (Epicentre Technologies). Lorsque le milieu réactionnel comporte les 4 NTP, l'ARN produit est dépourvu de coiffe (il est alors doté d'une extrémité 5' de type 5'pppGpNpN...). Pour obtenir l'ARN coiffé, le GTP est remplacé par un analogue de la coiffe, le m7G(5')ppp(5')G. Ce composé, reconnu par la polymérase, est incorporé à l'extrémité 5' du transcrit naissant lors de l'étape d'initiation de la transcription. Toutefois, il ne peut pas être incorporé lors de l'étape d'élongation. Aussi, l'ADN servant de matrice pour la synthèse de l'ARN a été conçu de telle sorte que le brin codant ne porte qu'une seule cytosine en position +1.

### 3. Oxydation de la coiffe en 5'

On dissout 0,1 OD d'ARN : on a utilisé deux oligoribonucléotides de 47 et 46 nucléotides dont 1 coiffé (+Cap, 47 nucléotides), et l'autre non coiffé (-Cap, 46 nucléotides). dans 9 µl de tampon acétate (0.1 M acétate de sodium pII 5.2) et 3 µl de solution de periodate de sodium 0.1 M préparée extemporanément.

On incube le mélange 1 heure à l'obscurité. Puis on arrête la réaction en ajoutant 4 µl d'éthylène glycol 10 %. On effectue une précipitation éthanolique du produit et on dialyse contre de l'eau.

### 4. Couplage du dialdéhyde avec de la biotine

On dissout le produit d'oxydation obtenu à l'étape précédente dans 50 µl d'acétate de sodium à pH entre 5 et 5.2 et 50 µl de solution d'hydrazide de biotine 0.02M préparée extemporanément dans un mélange méthoxyéthanol/eau (1:1) de formule :

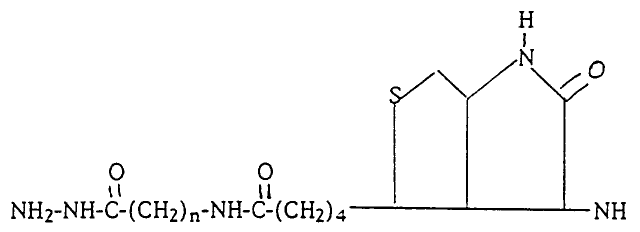

On a utilisé un espaceur au sein de l'hydrazide de biotine ayant n=5. On peut très bien utiliser les autres hydrazides disponibles dans le commerce : avec n variant de 0 à 5.

Puis on incube 2 heures à 37°C. On précipite à l'éthanol et on dialyse contre de l'eau distillée.

### EXEMPLE 2 : Capture de l'ARNm marqué avec des billes magnétiques

1. Les billes magnétiques revêtues de Streptavidine sont préparées selon les instructions du fournisseur (CPG Inc. USA). Les ARN sont ajoutés dans un tampon d'hybridation (NaCl 1,5M, pH 5-6). Puis après 30 minutes d'incubation le matériel non fixé et non biotinylé est éliminé. Les billes sont lavées plusieurs fois à l'eau avec 1% de SDS.

### 2. Récupération de l'ARNm

On incube les billes obtenues 15 minutes à 95°C dans de l'eau contenant 2% de SDS.

### 3. Résultats expérimentaux

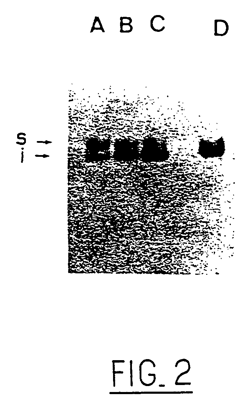

Dans les deux cas, figures 1 et 2, les résultats expérimentaux présentés correspondent à des autoradiogrammes de gel d'acrylamide 12 % de 0,4 mm d'épaisseur en tampon TBE.

La figure 1 montre les résultats obtenus avec des oligoribonucléotides de 47 nucléotides dont les séquences nucléotidiques correspondent aux séquences données ci-dessus (exemple 1) :

Colonnes A et B : oligonucléotide de 46 nucléotides sans coiffe marqué au pCp radioactif (exemple 1, 1.) soumis (B) ou non soumis (A) au procédé décrit en exemple 1, paragraphes 3.et 4. Colonnes C et D : oligonucléotide de 47 nucléotides avec coiffe marqué au pCp radioactif soumis (D) ou non soumis (C) au procédé décrit en exemple 1 (paragraphes 1. à 4.).

La différence de migration observée entre les colonnes A et C est dûe à la présence de la coiffe sur les oligonucléotides migrant dans la colonne C. La différence de migration observée entre les colonnes C et D est dûe à la biotynilation de la coiffe des oligonucléotides. Ceci démontre la spécificité de la biotynilation de la coiffe.

La Figure 2 montre les résultats obtenus lors de la récupération des oligonucléotides biotynilés et capturés par la streptavidine magnétique. L'oligonucléotide a été marqué au pCp radioactif en 3', puis soumis au procédé décrit dans l'exemple 1.1. Par la suite, les oligonucléotides biotynilés ont été capturés avec des billes magnétiques comme décrit dans l'exemple 2. Puis incubés, 5 (colonne A), 15 (colonne B) et 30 (colonne C) minutes à 95°C en présence de 2% SDS.

Les produits de la réaction ont été analysés par électrophorèse en gels de 12 % polyacrylamide en conditions dénaturantes (7M urée). Les gels ont subi une autoradiographie. Dans cette manipulation, les liaisons hydrazones n'ont pas été réduites.

Les bandes supérieures (flèche s) correspondent aux ARNs biotynilés avec la liaison hydrazone unissant le linker de la biotine et de l'oligoribonucléotide. Les bandes inférieures (flèche i) correspondent aux molécules (oligoribonucléotide) oxydées. La liaison hydrazone n'ayant pas été stabilisée au cours du chauffage, elle peut se casser et l'oligoribonucléotide retourne à la forme dialdéhyde. La colonne D présente l'oligoribonucléotide marqué au pCp comme décrit dans l'exemple 1.1.

### EXEMPLE 3 : METHODE DE PREPARATION D'ADNc COMPLETS . ETUDE DES ARNs COIFFES OU NON COIFFES SYNTHETIQUES

Cette méthode comprend les étapes de :
A) Marquage de l'extrémité 5' des ARNs par une étiquette
   A.1) Oxydation du diol de la méthyl-7-guanosine en dialdéhyde.
   A.2) Fonctionalisation de l'extrémité 3' de l'étiquette qui doit présenter un groupement NH2.
   A.3) Ligature chimique de l'étiquette fonctionnalisée sur la coiffe.
B) Transcription inverse des ARN étiquetés.

La transcription inverse est amorcée suivant l'une des voies classiques consistant à utiliser une amorce oligo-dT, une amorce aléatoire ou encore un amorçage spécifique comme dans les réactions d'extension d'amorces.

### 1) Méthode

### 1.1. Fonctionnalisation de l'oligonucléotide étiquette à ligaturer

L'acide nucléique phosphorylé en 3' est transformé en hydrazide en 3' par le traitement de l'acide nucléique avec une solution aqueuse d'hydrazine ou de dihydrazide de formule H2N(R1) NH2 à environ 1 à 3M, et à pH 4,5, en présence d'un agent du type carbodiimide soluble dans l'eau, par exemple du 1-ethyl-3 (3-dimethylaminopropyl) carbodiimide, à une concentration finale de 0.3M à une température de 8°C pendant la nuit.

L'oligonucléotide est alors séparé des autres agents et produits par une technique standard d'isolement des oligonucléotides.

### 1.2. Préparation de la molécule d'ARNm receveuse de l'étiquette.

### a) élimination des fonctions 3'-OH

- par ligature enzymatique de séquences dépourvues de 3'-OH par exemple ligature de pCp comme décrit dans (14) ;
- par hydrolyse alcaline des ARNs selon le protocole suivant dans lequel on réalise l'hydrolyse alcaline dans un volume final de 100 µl en présence de 1,5 µg d'ARNm en soude 0,1N durant 40 à 60 minutes à 4°C puis la solution est neutralisée à l'acide acétique et précipitée dans l'éthanol.

### b) Oxydation des fonctions diol.

. Jusqu'à 1 DO d'ARN est dissoute dans 9 µl de tampon (0.1 M acétate de sodium pH 6-7 ou d'eau) et 3 µl de solution de periodate de sodium 0.1M préparée extemporanément.
. On laisse incuber 1h à l'obscurité.
. On arrête la réaction en ajoutant 4 µl d'éthylène glycol 10%.
. On laisse incuber à température ambiante 15 minutes
. On fait une précipitation éthanolique du produit et on dialyse contre de l'eau.

### 1.3. Ligature chimique.

. On dissout l'ARN dans un milieu acide 50 µl d'acétate de sodium pH 4-6 et 50 µl de solution d'étiquette fonctionnalisée, puis on réduit avec un borohydrure, de telle sorte qu'un rapport 1:20 ARN:étiquette soit obtenu.
. On laisse incuber 2h00 à 37°C ou une nuit (14h) à 10°C.
. On précipite à l'éthanol et on dialyse contre de l'eau distillée pour les analyses comme des électrophorèses en gel d'acrylamide ou des HPLC.

### 1.4. Transcription inverse.

### a) synthèse du premier brin ADNc

Matériel et réactifs
- Bains Marie (90°C et 40°C)
- Carbo-glace
- Albumine de sérum bovin acetylé (10mg/ml (Biolab's)
- RNasine (5U/ µl, Promega Biotech). la RNAsine est fournie à la concentration de 40U/µl, on la dilue à 5U/µl dans un tampon RT
- β-mercaptoethanol (350mM Sigma). Le β-mercaptoethanol commercialisé est fourni à la concentration de 14,4M, pour obtenir une solution de 350mM µl mélanger 50 µl avec 1950 µl d'eau distillée.
- 10 x tampon RT (100 mM Tris HCL, ph: 8.3 (42°C), 80mM KCL, 16mM MgCL2)
- 100 mM de solutions de chaque dNTP (Pharmacia ou Boehringer)
- 1mM dCTP
- 20mM de pyrophosphate de sodium
- (α- 32P) dCTP 400 Ci/mmol (Amersham

L'oligonucléotide utilisé pour amorcer la transcriptase inversée en solution (100ng/µl en eau distillée) en eau distillée.
- oligo dT (15-17 (Boehringer)
- amorces à séquence aléatoire
- amorce spécifique

### Méthode :

- l'ARN est dilué dans 18 µl d'eau chauffée à 95°C, 5 min, puis on ajoute la solution décrite ci-dessus en 1.2.
- on mélange les composants suivants dans un tube à microcentrifugation

| | |
|---|---|
| 10 X tampon RT- | 2,5 µl |
| BSA (5 1-µl/ µl-) | 1 µl |
| RNAsine (5U/µl) | 1 µl |
| dATP(100mM) | 2 µl |
| dTTP(100mM) | 0,5 µl |
| dGTP (100mM) | 0,5 µl |
| dCTP (1mM) | 2,5 µl |
| pyrophosphate de sodium (200mM) | 1 µl |
| eau à 24 µl | |

On mélange avec la solution d'ARN avec l'amorce hybridée, on incube pendant 45 minutes à 42°C et,
On conserve à -20°C jusqu'à l'utilisation.

### 2) Résultats expérimentaux

### 2.1. Ligature d'oligodéoxyribonucléotides à 200 mère et un 46 mère ARN.

A) Des oligorionucléotides de 46 ou 200nt sont produits in vitro. En fonction des réactifs utilisés pour leur synthèse, ces oligoribonucléotides sont coiffés ou non (respectivement 200 ⁺, 46⁺ et 200⁻, 46⁻). Les séquences des oligonucléotides 46⁺ et 46⁻ ont été données dans l'exemple 1, paragraphe 3. Pour le 200 mère, les séquences sont les suivantes.

Après leur synthèse, les oligonucléotides sont soumis au procédé décrit en 1.2.a (ligture au pCp), pour bloquer leur extrémité 3'-OH puis au procédé décrit en 1.2.b pour oxyder les oligonucléotides coiffés.

B) Un oligodéoxyribonucléotide de 17 nt (GTTAGTGTGGTTGATCT) dont l'extrémité 3'OH a été modifiée en 3'-P est soumis au procédé décrit en 1.1. pour lui adjoindre une fonction hydrazide en 3'.

C) Par la suite, l'oligodéoxyribonucléotide produit en 2.1.B et les oligoribonucléotides produits en 2.1.A sont mis en ligature comme décrit au 1.3.

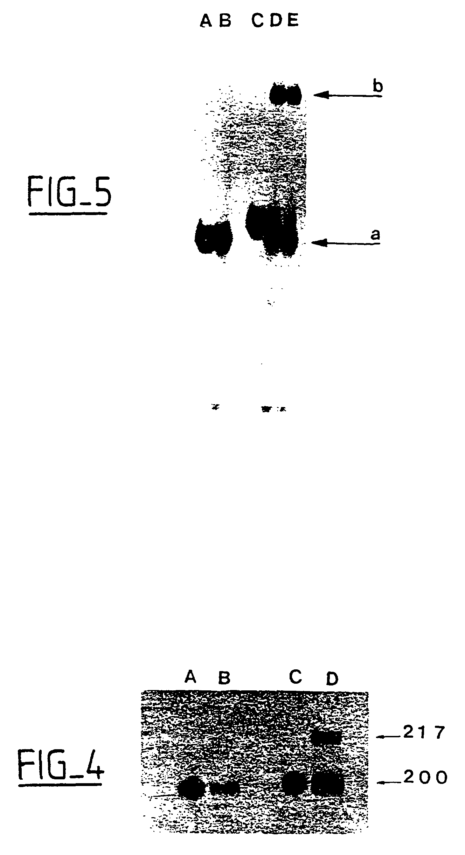

Figure 4 : dans les colonnes A et B sont représentés les résultats où sont déposés les oligoribonucléotides de 200nt non coiffés, ligaturés (colonne B) ou non (colonne A) avec un oligodésoxyribonucléotide hydraziné. Dans les colonnes C et D sont déposés les oligoribonucléotides de 200nt coiffés, soumis (colonne D) ou non (colonne C) à la ligature avec un oligodésoxyribonucléotide hydraziné.

Cet exemple montre la spécificité de la réaction d'étiquetage des ARNs coiffés. Dans les conditions expérimentales utilisées, 30 % de l'oligoribonucléotide coiffé sont ligaturés à l'étiquette.

Figure 5 : dans les colonnes A et B sont représentés les résultats où sont déposés les oligoribonucléotides de 46nt non coiffés ligaturés (colonne B) ou non (colonne A) avec un oligodésoxyribonucléotide hydraziné. Dans les colonnes C, D et E sont représentés les résultats où sont déposés les oligoribonucléotides coiffés ligaturés avec un oligodésoxyribonucléotide hydraziné (colonnes D et E). La colonne C représente l'oligoribonucléotide coiffé non oxydé et non ligaturé. Dans les colonnes D et E les bandes à la hauteur de la flèche a représenté l'oligoribonucléotide de 46nt oxydé non ligaturé, les bandes à la hauteur des flèches b représentent le produit de ligature.

Cet exemple montre la spécificité de la réaction d'étiquetage des ARNs coiffés avec une autre espèce moléculaire d'ARN.

### 2.2. Transcription inverse des produits ligaturés.

Un oligoribonucléotide de 46nt est produit in vitro, coiffé ou non. Puis, il est soumis au procédé de ligature à une étiquette comme décrit en 1.3, afin de le ligaturer à un oligodésoxyribonucléotide hydraziné de 17 nt.

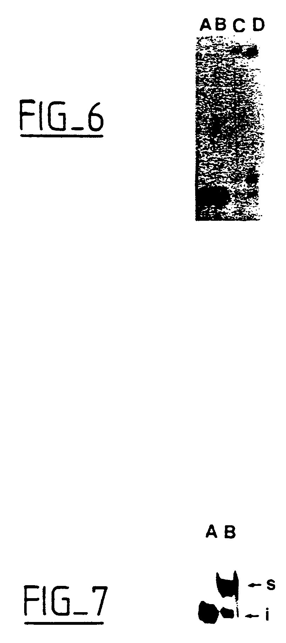

L'oligoribonucléotide ligaturé (correspondant aux bandes b des colonnes D et E, figure 5) et l'oligoribonucléotide non ligaturé (correspondant aux bandes a des colonnes D et E de la figure 5) ont été purifiés sur gel. Dans la figure 6 les colonnes A et B représentent l'oligoribonucléotide non ligaturé reverse transcrit (colonne B) ou non (colonne A). Les colonnes C et D représentent l'oligonucléotide ligaturé reverse transcrit (colonne D) ou non (colonne C).

On remarque que dans les deux cas, les produits sont radiomarqués car, le 46 mere produit est bloqué en 3' au pCp radioactif. Mais, les mêmes quantités d'oligonucléotides ont été déposées dans les colonnes A, B, C et D. Le signal plus intense dans les colonnes B et D ainsi que la présence d'une bande surnuméraire témoignent de la réalisation de la transcription inverse.

Le signal de la colonne D indique que la transcription inverse a été réalisée à travers la coiffe.

### EXEMPLE 4 : METHODE DE PREPARATION D'ADNc COMPLETS (II : ETUDE SUR DES ARNm PLACENTAIRES)

La méthode comprend les mêmes étapes que dans l'exemple 3. Nous avons apporté quelques modifications dans la réalisation pratique de la méthode.

### 1) Méthode

### 1.1. Fonctionalisation de l'oligonucléotide étiquette à ligaturer.

3 unité DO de l'oligodéoxyribonucléotide de séquence
ATCAAGAATTCGCACGAGACCATTA (extrémités 5' -OH et 3' -P) (seq. id n° 8) sont dissoutes dans 70 µl d'une solution 1.5 M d'hydroxybenzotriazole pH 5.3 réalisée dans du Dimethylformamide/Eau (75:25) contenant 2 µg de 1-ethyl-3 (3-dimethylamibnopropyl) carbodiimide. Incuber 2h30 à 22°C.

Précipiter deux fois dans du LiClO₄/Acétone
Resuspendre le culot dans 200µl d'hydrazine 0.25 M.
Incuber à 8°C de 3 à 14h00.
Précipiter deux fois dans du LiClO₄/Acétone.

### 1.2. Préparation de la molécule receveuse de l'étiquette.

### a) Préparation des ARNm de placenta

Les ARNs messagers ont été extraits à partir de blocs de 2cm de côté de placenta conservé à -80°C en utilisant les techniques classiques d'extraction d'ARN totaux en phénol acide puis de chromatographie oligo dT pour purifier les ARNm. L'intégrité des ARNm est vérifiée par Northern Blot.

### b) Oxydation des fonctions diols

Comme décrit dans l'exemple 3, paragraphe 1.2.
1.3. La Ligature chimique a été réalisée comme décrit dans l'exemple 3 paragraphe 1.3. Avec la modification suivante : l'étape de précipitation est remplacée par une étape de chromatographie d'exclusion pour élimier les oligodésoxyribonucléotides hydrazinés non ligaturés. Le protocole suivit est le suivant :

### a) Préparation de la colonne

On équilibre 10ml de gel AcA34 (BioSepra#230151) dans 50 ml de tampon (Tp : 10mM Tris pH 8.0, 300 mM NaCl, 1 mM EDTA, 0?05 % SDS).

On laisse sédimenter. On élimine le surnageant. Le gel est remis en suspension dans 50 ml de Tp.

On répéte cette étape 2 ou 3 fois.

On introduit une bille de verre (diamètre 3mm) dans une pipette jetable de 2ml (longeur 25cm).

On remplit la pipette de la suspension de gel jusqu'à ce que la hauteur de gel se stabilise à 1cm du haut de la pipette.

On équilibre alors la colonne avec 20ml de tampon d'équilibration (10mM Tris Hcl pH 7,4, 20 mM NaCl).

### b) Chromatographie

On mélange 10µl d'échantillon (ARN étiquetté) dans 39µl d'Urée 10M et 2µl de tampon bleu-glycérol (dissoudre 5mg de bleu de bromophenol dans 60 % de glycérol (v/v), filtrer avec un filtre de 0,45µm de diamètre).

On dépose sur la colonne. Dès que l'échantillon a pénétré, ajouter du tampon d'équilibration.

On collecte des fractions de 100µl. Une étiquette de 46nt apparaît dans la fraction 16 et les suivantes.

On conserve les fractions 3 à 15, les réunir puis les précipiter à l'éthanol.

### 1.4. Transcription inverse

La transcription inverse est réalisée avec la transcription inverse Superscript II de Gibco-BRL en suivant les instructions du fabricant. Pour amorcer la réaction 50 pmoles de nonamères à séquence aléatoire sont utilisés.

### 1.5. Empreintes d'ARN et d'ADNc

Les empreintes d'ADNc et d'ARN (RNA et cDNA-blots) ont été réalisées sur une membrane de nylon chargée positivement selon les méthodes classiquement utilisées. Les ADNc ont été déposés sur la membrane après que les hétéroduplexes ADNc:ARN aient subi une hydrolyse alcaline pour éliminer les ARNs.

Un oligodésoxyribonucléotide de séquence (TAATGGTCTCGTGCGAATTCTTGAT) (seq. id n° 9) anti-complémentaire de l'oligonucléotide ligaturé est marqué à son extrémité 5' avec du ³²p et hybridé avec les cDNA-blots.

### 1.6. Amplification en chaîne par polymérase (ACP).

Les ADNc obtenus après transcription inverse sont utilisés comme matrice de réaction d'ACP. Deux types de réactions sont réalisés.
a) Des amplifications pour détecter des séquences spécifiques (globine, deshydrogenase, pp15 et facteur d'élongation E4). Les amorces oligodesoxyribonucléotidiques suivantes ont été utilisées.
   alpha-globine deshydrogenase pp15 Facteur d'élongation E4
b) Des amplifications réalisées avec les oligodesoxyribonucléotides antisens (_As) des couples décrits précédemment et une amorce choisit dans la séquence de l'oligodesoxyribonucléotide ligaturé (ATCAAGAATTCGCACGAGACCATTA).

### 2) Résultats expérimentaux

### 2.1. Efficacité de la ligature chimique

L'oligonucléotide de 25nt décrit au 1.1. Exemple 4 a été modifié comme décrit dans ce paragraphe et ligaturé à l'oligoribonucléotide de 200nt coiffé ou non décrit dans l'exemple 3.

Figure 7 : Colonne A : oligoribonucléotide de 200nt (radiomarqué à son extrémité 3' par du ³²pCp) non coiffé, oxydé et ligaturé à l'étiquette. Colonne B : oligoribonucléotide de 200nt (radiomarqué à son extrémité 3' par du ³²pCp) coiffé oxydé et ligaturé à l'étiquette hydrazinée. Dans cette colonne la bande inférieure (i) correspond au 200 mère non ligaturé tandis que la bande supérieure (s) correspond au 200 mère ligaturé. Dans les conditions de préparation de l'étiquette et de ligature, 80 % de l'oligonucléotide coiffé ont été ligaturés.

### 2.2 Ligature de l'étiquette sur des ARN poly A⁺.

Des ARNs messagers placentaires (7µg) sont oxydés dans les conditions décrites dans l'exemple 3 puis ligaturés à un olidésoxyribonucléotide hydraziné dans les conditions décrites dans l'exemple 4. L'oligonucléotide non ligaturé est éliminé au moyen d'une chromatographie d'exclusion (AcA34) comme décrit au point 1.3 de l'exemple 4. La présence d'étiquette ligaturée est vérifiée par hybridation d'une sonde avec des dépôts d'ARNs étiquetés sur une membrane de Nylon comme décrit au point 1.5. de l'exemple 4.

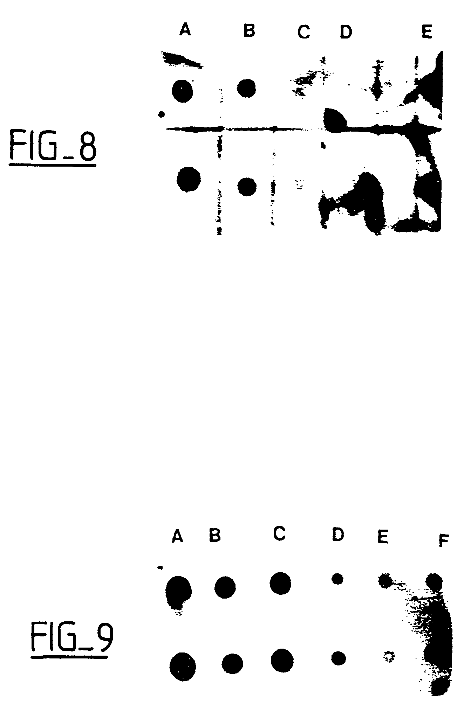

La figure 8 représente une autoradiographie de la membrane après hybridation de la sonde. Tous les points sont dupliqués. Colonnes A à D : 0.5, 5, 25 et 50 fmôles d'étiquette. Colonne E : 1/10ème des ARNm étiquettés ont été déposés en deux dépôts.

### 2.3. Transcription inverse à travers la coiffe

Les 9/10ème des ARNs étiquettés obtenus au point 2.2. de l'exemple 4 sont réverse transcrits comme décrits en 1.4, Exemple 4, puis déposés sur membrane et analysés comme décrit en 1.6, Exemple 4, ou utilisés comme matrice de réactions d'ACP comme décrit en 1.6 a) et b).

La figure 9 montre une autoradiographie de la membrane après hybridation avec la sonde radiomarquée décrite en 1.6.

Les colonnes A à E représentent différentes concentrations (1 pmôle, 100 fmôles, 50 fmôles, 10 fmôles et 1 fmôle) d'un oligodésoxyribonucléotide témoin de séquence identique à celle de l'étiquette ligaturée. Colonne F : Dépôt des ADNc-simple brin étiquettés. Approximativement 15 fmoles d'étiquette sont présentes.

Ces résultats démontrent que la transcription inverse peut être réalisée à travers la coiffe et en particulier que la transcriptase inverse franchie la liaison 5'-P-P-P-5' de la coiffe des ARN messagers eucaryotes.

La figure 10 représente la photographie d'un gel d'agarose 1,5 % coloré au bromure d'éthidium des produits d'ACP de la transcription inverse (1/20ème des produits de transcription inverse ont été utilisés pour chaque réaction d'ACP).

Colonnes A et B : marqueurs de poids moléculaires (décrire). Colonnes C et D : ACP avec les amorces globine en présence d'ADNc (Colonne C) ou non (Colonne D). Colonnes E et F : ACP avec les amorces deshydrogenase en présence d'ADNc (colonne E) ou non (colonne F). Colonnes G et H : ACP avec les amorces pp15 en présence (Colonne G) ou non (colonne H) d'ADNc. Colonnes I et J : ACP avec les amorces EIE4 en présence (colonne I) ou non (colonne J) d'ADNc.

Dans les colonnes C, E, G et I la présence d'une bande à la taille attendue témoigne de la présence de la séquence correspondante dans la population d'ADNc.

La figure 11 représente la photographie d'un gel d'agarose 1,5 % coloré au bromure d'éthidium. Même légende que la figure 10, mais les ACP ont été réalisées avec les oligonucléotides anti sens de chaque couple d'amorce et un oligonucléotide dont la séquence correspond à celle de l'étiquette ligaturé. Les produits d'amplification des colonnes C et E montrent que la transcription inverse a recopié la molécule d'ARN et l'étiquette oligodesoxyribonucléotidique ligaturée. Les profils obtenus dans la colonne C sont vraisemblablement dûs aux conditions d'ACP qui n'ont pas été optimisées pour les couples d'amorces utilisés.

### BIBLIOGRAPHIE

1. Frohman et Coll. (1988), PNAS 85, 8998-9002 ;
2. Delort et Coll. (1989), NAR 17, 6439-6448 ;
3. Dumas Milne Edwards et Coll. (1991) NAR 19, 5227-5232 ;
4. Frohman-Racine (1993), NAR 21, 1683-1684 ;
5. Maruyama et Sugano (1993) Gene 138, 171-174 ;
6. Dumas Milne Edwards (1993): Thèse de Docterat, Paris VI, 75-90 ;
7. Kato et Sekine (1993), EPO-Application : #93921061.3 ;
8. Sonenberg et Altmann (1989), EPO-Application : #89313030.2 ;
9. Reddy et Coll. (1992), Pharmac. Ther. Vol 54, pp. 249-267 ;
10. Zamecnik et Coll. (1960), PNAS 46, 811 ;
11. Agrawal et Coll. (1986), NAP 14, 6227-6245 ;
12. O'Shannessy D.J. et Coll. (1987), Anal. Biochem. 163, 204-209 ;
13. Ghosh F., EPO application N° 89 309 532.3;
14. Uhlenbeck O.C., Gumport R.J. (1982), T4 RNA Ligase, The Enzymes, V. 15B (P.D. Boyer ed) pp 31-60. Academic Press ;
15. Current Protocol in Molecular Biology. N.Y. 3.15.1. (1994) Ed. John Wiley and Sons, Inc ;

## Revendications

1. Méthode pour obtenir un polynucléotide comprenant la région 5' d'un ARNm ou d'un fragment d'ARNm à partir d'une population d'ARNm eucaryotes comprenant les étapes de :
a) oxyder un 2',3'-cis -diol sur une coiffe située à l'extrémité 5' dudit ARNm ou dudit fragment ARNm pour générer un dialdéhyde ;
b) coupler ledit dialdéhyde à un groupe fonctionnel amine sur une molécule de couplage pour former un conjugé qui comprend ladite molécule de couplage et ledit polynucléotide ; et
c) isoler ledit conjugé de ladite population d'ARNm.

2. Méthode selon la revendication 1 comprenant en outre l'étape d'effectuer une réaction de transcription inverse sur ledit polynucléotide.

3. Méthode selon la revendication 1 comportant en outre l'étape de modifier la base à l'extrémité 3' dudit ARNm ou fragment d'ARNm avant d'effectuer ladite étape d'oxydation de telle sorte que ladite base ne possède pas de groupe OH en position 2' et 3'.

4. Méthode selon la revendication 3 dans laquelle ladite étape de modification comprend l'addition d'un nucléotide ou d'un oligonucléotide dont l'extrémité 3' ne comprend pas de groupe fonctionnel 2',3'- diol à ladite base.

5. Méthode selon la revendication 4, dans laquelle ladite étape de modification comprend la ligation d'un nucléoside 3', 5'-diphosphate (pNp) à ladite base en utilisant une enzyme.

6. Méthode selon la revendication 5, dans laquelle ladite enzyme consiste en l'ARN ligase T4.

7. Méthode selon la revendication 3, dans laquelle ladite étape de modification comprend :
a) l'étape d'effectuer une hydrolyse alcaline ménagée sur des ARNm ou des fragments d'ARNm contenant un groupe fonctionnel 2',3'-diol à l'extrémité 3' de leurs queues polyA afin de générer des fragments d'ARNm ne contenant plus ledit groupe fonctionnel 2',3'-diol à leur extrémité 3', et
b) l'étape de séparer lesdits fragments d'ARNm ne contenant plus ledit groupe fonctionnel 2',3'-diol à leur extrémité 3' des fragments d'ARNm qui contiennent ledit groupe fonctionnel 2',3'-diol à leur extrémité 3'.

8. Méthode selon la revendication 1, dans laquelle ladite étape d'oxydation comprend l'étape d'effectuer une réaction d'oxydation avec un périodate.

9. Méthode selon la revendication 1, dans laquelle ladite étape de couplage est faite dans un milieu acide et ladite méthode comprend de plus l'étape d'effectuer une réaction de réduction avec un borohydrure.

10. Méthode selon la revendication 1, dans laquelle ladite molécule de couplage est choisie dans le groupe suivant :
a) hydrazide de biotine,
b) une molécule qui ne se lie pas naturellement à ladite coiffe ;
c) une molécule marquée ; et
d) une molécule qui comprend une molécule biologique.

11. Méthode selon la revendication 1, dans laquelle ladite molécule de couplage comprend une molécule qui facilite l'isolation dudit ARNm ou fragment d'ARNm.

12. Méthode selon la revendication 11, dans laquelle ladite étape de couplage comprend de plus les étapes de :
a) mettre en contact ledit conjugué avec une seconde molécule, se liant de façon covalente ou non covalente à ladite molécule de couplage, et formant ainsi un complexe qui comprend ladite seconde molécule, ladite molécule de couplage, et ledit ARNm ou fragment d'ARNm; et
b) séparer ledit complexe desdits ARNm ou fragments d'ARNm qui n'ont pas formé ledit complexe.

13. Méthode selon la revendication 12, dans laquelle ledit ARNm comprend un ARNm complet.

14. Méthode selon la revendication 12, comprenant en outre l'étape de cliver la liaison entre ladite seconde molécule et ladite molécule de couplage dans ledit complexe, libérant ainsi ledit conjugué.

15. Méthode selon la revendication 14, comprenant en outre l'étape de cliver ledit conjugué, générant ainsi un ARNm ou fragment d'ARNm non conjugué.

16. Méthode selon la revendication 12, dans laquelle ladite molécule de couplage et ladite seconde molécule sont choisies dans le groupe consistant de :
a) des molécules biologiques qui s'associent pour former un complexe non covalent ; et
b) de l'hydrazide de biotine pour la molécule de couplage et soit la streptavidine ou l'avidine pour la seconde molécule.

17. Méthode selon la revendication 12, dans laquelle ladite seconde molécule s'attache à une phase solide et ladite étape de séparation comprend la séparation de ladite phase solide desdits ARNm ou fragments d'ARNm qui n'ont pas formé ledit complexe.

18. Méthode selon la revendication 17, dans laquelle ladite phase solide est choisie dans le groupe consistant de la face interne d'une cuve dans laquelle lesdits ARNm ou fragments d'ARNm sont présents et de composants qui sont introduits dans ladite cuve.

19. Polynucléotide isolé qui comprend un conjugué ARNm-molécule de couplage obtenu par la méthode de la revendication 1 ou de la revendication 12.

20. Méthode pour synthétiser un premier brin d'ADNc, comprenant les étapes de :
a) oxyder les 2',3'-cis -diols sur les coiffes situées aux extrémités 5' des polynucléotides comprenant les ARNm eucaryotes ou des fragments d'ARNm eucaryotes pour générer des dialdéhydes ;
b) coupler lesdits dialdéhydes à des groupes fonctionnels amine sur des molécules de couplage pour former des conjugués qui comprennent lesdites molécules de couplage et lesdits polynucléotides ;
c) effectuer une réaction de transcription inverse sur lesdits conjugués formant ainsi des hétéroduplexes qui comprennent un premier brin ADNc et lesdits ARNm ou fragments d'ARNm dans lesdits conjugués ;
d) éliminer les ARNm ou les fragments d'ARNm qui n'ont pas formé lesdits hétéroduplexes ;
e) capturer lesdits hétéroduplexes sur une phase solide revêtue avec une seconde molécule capable de se lier à ladite molécule de couplage ; et
f) dénaturer lesdits hétéroduplexes pour récupérer ledit premier brin ADNc.

21. Méthode selon la revendication 20, dans laquelle ladite méthode d'élimination comprend la digestion avec une enzyme qui dégrade les ARNm ou fragments d'ARNm simples brins ou non hybridés tout en laissant les hétéroduplexes intacts.

22. Méthode selon la revendication 21 dans laquelle ladite enzyme est choisie dans le groupe consistant de la RNAse T1 et de la nucléase S1.

23. Méthode pour synthétiser des ADNc double brins correspondant aux extrémités 5' de polynucléotides qui comprennent des ARNm eucaryotes ou des fragments d'ARNm eucaryotes comportant les étapes de :
a) oxyder les 2',3'-cis-diols sur les coiffes situées auxdites extrémités 5' desdits ARNm ou fragments d'ARNm pour générer des dialdéhydes ;
b) coupler un groupe fonctionnel amine de l'extrémité 3' d'un oligonucléotide auxdits dialdéhydes formant ainsi des conjugués oligonucléotides-ARNm ;
c) effectuer une réaction de transcription inverse sur ledit conjugué oligonucléotide-ARNm, formant ainsi des hétéroduplexes qui comprennent des premiers brins d'ADNc et lesdits conjugués oligonucléotides-ARNm ;
d) dénaturer lesdits hétéroduplexes ;
e) éliminer les ARNm ou fragments d'ARNm simple brin ; et
f) effectuer la synthèse des deuxièmes brins d'ADNc complémentaires auxdits premiers brins d'ADNc en utilisant une amorce complémentaire à tout ou partie de la séquence dudit oligonucléotide.

24. Méthode selon la revendication 23, dans laquelle les ADNc obtenus correspondent à des ARNm complets.

25. Méthode pour obtenir une protéine capable de se lier à un ARNm eucaryotes comprenant les étapes de :
a) coupler un groupe fonctionnel amine d'une molécule de couplage à la coiffe située à l'extrémité 5' d'un polynucléotide comprenant un ARNm ou un fragment d'ARNm formant ainsi un conjugué molécule de couplage-ARNm ;
b) mettre en contact ledit conjugué molécule de couplage-ARNm avec une protéine capable de reconnaître ledit ARNm ou fragment d'ARNm, formant ainsi un complexe comprenant ladite protéine et ledit conjugué molécule de couplage-ARNm ;
c) isoler ledit complexe ;
d) dissocier ladite protéine dudit complexe molécule de couplage-ARNm ; et
e) récupérer ladite protéine.

26. Kit pour l'obtention d'un polynucléotide comprenant la région 5' d'un ARNm ou d'un fragment d'ARNm à partir d'une population d'ARNm eucaryotes comprenant:
a) un réactif de modification pour modifier la base à l'extrémité 3' dudit ARNm ou fragment d'ARNm de telle sorte que ladite base n'ait plus de groupe OH en position 2' et 3' ;
b) un réactif d'oxydation pour oxyder un 2'-3'-cis-diol sur des coiffes situées aux extrémités 5' desdits ARNm ou fragments d'ARNm pour générer des dialdéhydes ;
c) une molécule de couplage possédant un groupe fonctionnel amine pour former un conjugué comprenant ladite molécule de couplage et ledit polynucléotide ;
d) un réactif de couplage pour coupler ledit dialdéhyde audit groupe fonctionnel amine.

27. Kit selon la revendication 26, dans lequel ledit réactif d'oxydation est le périodate de sodium.

28. Kit selon la revendication 26, dans lequel ledit réactif de couplage comprend un tampon acide qui a un pH entre 4 et 6 et un borohydrure.

29. Kit selon la revendication 26, dans lequel ladite molécule de couplage comprend une molécule choisie parmi le groupe suivant :
a) une molécule biologique ;
b) une molécule soluble ;
c) l'hydrazide de biotine ; et
d) un oligonucléotide possédant un groupe fonctionnel amine à son extrémité 3'.

30. Kit selon la revendication 26, dans lequel ledit réactif de modification comprend un réactif pour effectuer le couplage de l'extrémité 3' dudit ARNm ou fragment d'ARNm à l'extrémité 5' d'un oligonucléotide ou d'un nucléotide, dans lequel ledit oligonucléotide ou nucléotide ne possède pas de 2',3'-cis-diol à son extrémité 3'.

31. Kit selon la revendication 30, dans lequel ledit réactif de modification comprend un nucléotide 3',5'-diphosphate pNp et une enzyme de ligation.

32. Kit selon la revendication 31, dans lequel ladite enzyme de ligation consiste en l'ARN ligase T4.

33. Kit selon la revendication 26, comprenant en outre des réactifs pour effectuer une réaction de transcription inverse.

34. Kit pour l'obtention d'un polynucléotide comprenant la région 5' d'un ARNm ou fragment d'ARNm à partir d'une population d'ARNm eucaryotes comprenant:
a) un réactif d'oxydation pour oxyder le 2',3'-cis-diol sur une coiffe située aux extrémités 5' desdits ARNm ou fragments d'ARNm pour générer un dialdéhyde ;
b) une molécule de couplage possédant un groupe fonctionnel amine afin de former un conjugué comprenant ladite molécule de couplage et ledit polynucléotide ;
c) un réactif de couplage pour effectuer le couplage covalent dudit dialdéhyde audit groupe fonctionnel amine ; et
d) une seconde molécule qui se lie de façon covalente ou non covalente à ladite molécule de couplage afin de former un complexe avec ladite molécule de couplage, ladite seconde molécule étant en solution ou attachée à une phase solide.

35. Kit selon la revendication 34, comprenant en outre un réactif de modification pour modifier la base à l'extrémité 3' dudit ARNm ou fragment d'ARNm de telle sorte que ladite base ne possède pas de groupe OH à ses positions 2' et 3'.

36. Kit de réactifs pour synthétiser des ADNc double brins correspondant aux extrémités 5' de polynucléotides comprenant des ARNm eucaryotes ou fragments d'ARNm eucaryotes comprenant :
a) un réactif d'oxydation afin d'oxyder le 2',3'-cis-diol sur une coiffe située aux extrémités 5' desdits ARNm afin de générer un dialdéhyde ;
b) un oligonucléotide contenant un groupe fonctionnel amine à son extrémité 3' ;
c) un réactif de couplage pour coupler de façon covalente ledit dialdéhyde audit oligonucléotide ;
d) une amorce et une enzyme pour effectuer une réaction de transcription inverse sur lesdits ARNm ou fragments d'ARNm couplés audit oligonucléotide afin de générer des hétéroduplexes comprenant un premier brin d'ADNc et ledit ARNm ou fragments d'ARNm couplés audit oligonucléotide ;
e) des réactifs pour dénaturer lesdits hétéroduplexes et éliminer lesdits ARNm ou fragments d'ARNm de telle sorte de récupérer seulement lesdits premiers brins d'ADNc ;
f) une amorce et une enzyme permettant la synthèse d'un deuxième brin d'ADNc.

## Claims

1. Method for obtaining a polynucleotide comprising the 5' region of an mRNA or of an mRNA fragment, from a population of eukaryotic mRNAs, comprising the steps of:
a) oxidizing a 2',3'-cis-diol on a cap located at the 5' end of said mRNA or of said mRNA fragment, so as to generate a dialdehyde;
b) coupling said dialdehyde to an amine functional group on a coupling molecule, so as to form a conjugate which comprises said coupling molecule and said polynucleotide; and
c) isolating said conjugate from said mRNA population.

2. Method according to Claim 1, also comprising the step of carrying out a reverse transcription reaction on said polynucleotide.

3. Method according to Claim 1, also comprising the step of modifying the base at the 3' end of said mRNA or mRNA fragment, before carrying out said oxidation step, such that said base does not have an OH group in the 2' and 3' position.

4. Method according to Claim 3, in which said modification step comprises adding a nucleotide or an oligonucleotide, the 3' end of which does not comprise a 2',3'-diol functional group, to said base.

5. Method according to Claim 4, in which said modification step comprises ligating a nucleoside 3',5'-diphosphate (pNp) to said base using an enzyme.

6. Method according to Claim 5, in which said enzyme consists of T4 RNA ligase.

7. Method according to Claim 3, in which said modification step comprises:
a) the step of carrying out a controlled alkaline hydrolysis on mRNAs or mRNA fragments, containing a 2',3'-diol functional group at the 3' end of their polyA tails, in order to generate mRNA fragments which no longer contain said 2',3'-diol functional group at their 3' end, and
b) the step of separating said mRNA fragments which no longer contain said 2',3'-diol functional group at their 3' end from the mRNA fragments which contain said 2',3'-diol functional group at their 3' end.

8. Method according to Claim 1, in which said oxidation step comprises the step of carrying out an oxidation reaction with a periodate.

9. Method according to Claim 1, in which said coupling step is carried out in an acid medium and said method also comprises the step of carrying out a reduction reaction with a borohydride.

10. Method according to Claim 1, in which said coupling molecule is chosen from the following group:
a) biotin hydrazide,
b) a molecule which does not naturally bind to said cap;
c) a labelled molecule; and
d) a molecule which comprises a biological molecule.

11. Method according to Claim 1, in which said coupling molecule comprises a molecule which facilitates the isolation of said mRNA or mRNA fragment.

12. Method according to Claim 11, in which said coupling step also comprises the steps of:
a) bringing said conjugate into contact with a second molecule, which binds covalently or non-covalently to said coupling molecule, and thus forms a complex which comprises said second molecule, said coupling molecule, and said mRNA or mRNA fragment; and
b) separating said complex from said mRNAs or mRNA fragments which have not formed said complex.

13. Method according to Claim 12, in which said mRNA comprises a complete mRNA.

14. Method according to Claim 12, also comprising the step of cleaving the bond between said second molecule and said coupling molecule in said complex, thus releasing said conjugate.

15. Method according to Claim 14, also comprising the step of cleaving said conjugate, thus generating a non-conjugated mRNA or mRNA fragment.

16. Method according to Claim 12, in which said coupling molecule and said second molecule are chosen from the group consisting of:
a) biological molecules which associate to form a non-covalent complex; and
b) biotin hydrazide for the coupling molecule, and either streptavidin or avidin for the second molecule.

17. Method according to Claim 12, in which said second molecule attaches to a solid phase and said separation step comprises separating said solid phase from said mRNAs or mRNA fragments which have not formed said complex.

18. Method according to Claim 17, in which said solid phase is chosen from the group consisting of the inner face of a tank in which said mRNAs or mRNA fragments are present and of components which are introduced into said tank.

19. Isolated polynucleotide which comprises an mRNA-coupling molecule conjugate obtained using the method of Claim 1 or Claim 12.

20. Method for synthesizing a first cDNA strand, comprising the steps of:
a) oxidizing the 2',3'-cis-diols on the caps located at the 5' ends of the polynucleotides comprising eukaryotic mRNAs or eukaryotic mRNA fragments, so as to generate dialdehydes;
b) coupling said dialdehydes to amine functional groups on coupling molecules, so as to form conjugates which comprise said coupling molecules and said polynucleotides;
c) carrying out a reverse transcription reaction on said conjugates, thus forming heteroduplexes which comprise a first cDNA strand and said mRNAs or mRNA fragments in said conjugates;
d) eliminating the mRNAs or the mRNA fragments which have not formed said heteroduplexes;
e) capturing said heteroduplexes on a solid phase coated with a second molecule capable of binding to said coupling molecule; and
f) denaturing said heteroduplexes so as to recover said first cDNA strand.

21. Method according to Claim 20, in which said elimination method comprises digestion with an enzyme which degrades the single-stranded or non-hybridized mRNAs or mRNA fragments, while at the same time leaving the heteroduplexes intact.

22. Method according to Claim 21, in which said enzyme is chosen from the group consisting of T1 RNAse and S1 nuclease.

23. Method for synthesizing double-stranded cDNAs corresponding to the 5' ends of polynucleotides which comprise eukaryotic mRNAs or eukaryotic mRNA fragments, comprising the steps of:
a) oxidizing the 2',3'-cis-diols on the caps located at said 5' ends of said mRNAs or mRNA fragments, so as to generate dialdehydes;
b) coupling an amine functional group of the 3' end of an oligonucleotide to said dialdehydes, thus forming oligonucleotide-mRNA conjugates;
c) carrying out a reverse transcription reaction on said oligonucleotide-mRNA conjugate, thus forming heteroduplexes which comprise first cDNA strands and said oligonucleotide-mRNA conjugates;
d) denaturing said heteroduplexes;
e) eliminating the single-stranded mRNAs or mRNA fragments; and
f) carrying out the synthesis of the second cDNA strands complementary to said first cDNA strands, using a primer complementary to all or part of the sequence of said oligonucleotide.

24. Method according to Claim 23, in which the cDNAs obtained correspond to complete mRNAs.

25. Method for obtaining a protein capable of binding to a eukaryotic mRNA, comprising the steps of:
a) coupling an amine functional group of a coupling molecule to the cap located at the 5' end of a polynucleotide comprising an mRNA or an mRNA fragment, thus forming a coupling molecule-mRNA conjugate;
b) bringing said coupling molecule-mRNA conjugate into contact with a protein capable of recognizing said mRNA or mRNA fragment, thus forming a complex comprising said protein and said coupling molecule-mRNA conjugate;
c) isolating said complex;
d) dissociating said protein from said coupling molecule-mRNA complex; and
e) recovering said protein.

26. Kit for obtaining a polynucleotide comprising the 5' region of an mRNA or of an mRNA fragment, from a population of eukaryotic mRNAs, comprising:
a) a modifying reagent for modifying the base at the 3' end of said mRNA or mRNA fragment, such that said base no longer has an OH group in the 2' and 3' position;
b) an oxidizing reagent for oxidizing a 2',3'-cis-diol on caps located at the 5' ends of said mRNAs or mRNA fragments, so as to generate dialdehydes;
c) a coupling molecule which has an amine functional group for forming a conjugate comprising said coupling molecule and said polynucleotide;
d) a coupling reagent for coupling said dialdehyde to said amine functional group.

27. Kit according to Claim 26, in which said oxidizing reagent is sodium periodate.

28. Kit according to Claim 26, in which said coupling reagent comprises an acid buffer which has a pH of between 4 and 6, and a borohydride.

29. Kit according to Claim 26, in which said coupling molecule comprises a molecule chosen from the following group:
a) a biological molecule;
b) a soluble molecule;
c) biotin hydrazide; and
d) an oligonucleotide which has an amine functional group at its 3' end.

30. Kit according to Claim 26, in which said modifying reagent comprises a reagent for carrying out the coupling of the 3' end of said mRNA or mRNA fragment to the 5' end of an oligonucleotide or of a nucleotide, in which said oligonucleotide or nucleotide does not have a 2',3'-cis-diol at its 3' end.

31. Kit according to Claim 30, in which said modifying reagent comprises a nucleotide 3',5'-diphosphate pNp and a ligating enzyme.

32. Kit according to Claim 31, in which said ligating enzyme consists of T4 RNA ligase.

33. Kit according to Claim 26, also comprising reagents for carrying out a reverse transcription reaction.

34. Kit for obtaining a polynucleotide comprising the 5' region of an mRNA or mRNA fragment, from a population of eukaryotic mRNAs, comprising:
a) an oxidizing reagent for oxidizing the 2',3'-cis-diol on a cap located at the 5' ends of said mRNAs or mRNA fragments, so as to generate a dialdehyde;
b) a coupling molecule which has an amine functional group in order to form a conjugate comprising said coupling molecule and said polynucleotide;
c) a coupling reagent for carrying out the covalent coupling of said dialdehyde to said amine functional group; and
d) a second molecule which binds covalently or non-covalently to said coupling molecule in order to form a complex with said coupling molecule, said second molecule being in solution or attached to a solid phase.

35. Kit according to Claim 34, also comprising a modifying reagent for modifying the base at the 3' end of said mRNA or mRNA fragment, such that said base does not have an OH group at its 2' and 3' positions.

36. Kit of reagents for synthesizing double-stranded cDNAs corresponding to the 5' ends of polynucleotides comprising eukaryotic mRNAs or eukaryotic mRNA fragments, comprising:
a) an oxidizing reagent in order to oxidize the 2',3'-cis-diol on a cap located at the 5' ends of said mRNAs in order to generate a dialdehyde;
b) an oligonucleotide containing an amine functional group at its 3' end;
c) a coupling reagent for covalently coupling said dialdehyde to said oligonucleotide;
d) a primer and an enzyme for carrying out a reverse transcription reaction on said mRNAs or mRNA fragments coupled to said oligonucleotide, in order to generate heteroduplexes comprising a first cDNA strand and said mRNA or mRNA fragments coupled to said oligonucleotide;
e) reagents for denaturing said heteroduplexes and eliminating said mRNAs or mRNA fragments, so as to recover only said first cDNA strands;
f) a primer and an enzyme for synthesizing a second cDNA strand.

## Patentansprüche

1. Verfahren zur Herstellung eines Polynucleotids mit der 5'-Region einer mRNA oder eines mRNA-Fragments von einer Eukaryonten-mRNA-Population, umfassend die folgenden Schritte:
a) Oxidation eines 2',3'-cis-Diols an einer am 5'-Ende dieser mRNA bzw. dieses mRNA-Fragments gelegenen Cap-Struktur, wodurch man zu einem Dialdehyd gelangt;
b) Koppeln des Dialdehyds mit einer funktionellen Aminogruppe an einem Kopplungsmolekül, wodurch man zu einem Konjugat gelangt, das das Kopplungsmolekül und das Polynucleotid enthält; sowie
c) Isolation des Konjugats aus der mRNA-Population.

2. Verfahren nach Anspruch 1, umfassend weiterhin den Schritt, daß man das Polynucleotid revers transkribiert.

3. Verfahren nach Anspruch 1, umfassend weiterhin den Schritt, daß man vor dem Oxidationsschritt die Base am 3'-Ende der mRNA bzw. des mRNA-Fragments so modifiziert, daß diese Base keine OH-Gruppe in 2'- und 3'-Stellung aufweist.

4. Verfahren nach Anspruch 3, bei dem der Modifikationsschritt die Addition eines Nucleotids oder eines Oligonucleotids, dessen 3'-Ende keine funktionelle 2',3'-Diolgruppe enthält, an diese Base umfaßt.

5. Verfahren nach Anspruch 4, bei dem der Modifikationsschritt die Ligation eines 3',5'-Diphosphatnucleosids (pNp) an diese Base mittles eines Enzyms umfaßt.

6. Verfahren nach Anspruch 5, bei dem das Enzym aus T4-RNA-Ligase besteht.

7. Verfahren nach Anspruch 3, bei dem der Modifikationsschritt folgendes umfaßt:
a) den Schritt, daß man mRNAs bzw. mRNA-Fragmente, die am 3'-Ende ihrer polyA-Schwänze eine funktionelle 2',3'-Diolgruppe enthalten, alkalisch hydrolysiert, wodurch man zu mRNA-Fragmenten gelangt, die an ihrem 3'-Ende keine funktionelle 2',3'-Diolgruppe mehr aufweisen, sowie
b) den Schritt, daß man diese mRNA-Fragmente, die an ihrem 3'-Ende diese funktionelle 2',3'-Diolgruppe nicht mehr aufweisen, von mRNA-Fragmenten, die an ihrem 3'-Ende diese funktionelle 2',3'-Diolgruppe aufweisen, trennt.

8. Verfahren nach Anspruch 1, bei dem der Oxidationsschritt umfaßt, daß man mit einem Periodat eine Oxidation durchführt.

9. Verfahren nach Anspruch 1, bei dem der Kopplungsschritt in einem sauren Medium durchgeführt wird und das Verfahren weiterhin den Schritt umfaßt, daß man mit einem Borhydrid eine Reduktion durchführt.

10. Verfahren nach Anspruch 1, bei dem das Kopplungsmolekül aus der Gruppe:
a) Biotinhydrazid,
b) ein Molekül, das mit dieser Cap-Struktur keine natürliche Bindung eingeht,
c) ein markierten Molekül sowie
d) ein Molekül, das ein biologisches Molekül umfaßt,
stammt.

11. Verfahren nach Anspruch 1, bei dem das Kopplungsmolekül ein Molekül umfaßt, das die Isolation der mRNA bzw. des mRNA-Fragments erleichtert.

12. Verfahren nach Anspruch 11, bei dem der Kopplungsschritt weiterhin die folgenden Schritte umfaßt:
a) In-Kontakt-bringen des Konjugats mit einem zweiten Molekül, das kovalent oder nichtkovalent an das Kopplungsmolekül bindet und so einen Komplex bildet, der das zweite Molekül, das Kopplungsmolekül, sowie die mRNA bzw. das mRNA-Fragment umfaßt, sowie
b) Abtrennung des Komplexes von den mRNAs bzw. mRNA-Fragmenten, die diesen Komplex nicht gebildet haben.

13. Verfahren nach Anspruch 12, bei dem die mRNA eine vollständige mRNA umfaßt.

14. Verfahren nach Anspruch 12, umfassend weiterhin den Schritt, daß man die Bindung zwischen dem zweiten Molekül und dem Kopplungsmolekül in dem Komplex spaltet und so das Konjugat freisetzt.

15. Verfahren nach Anspruch 14, umfassend weiterhin den Schritt, daß man das Konjugat spaltet, wodurch man zu einer nicht konjugierten mRNA bzw. einem nicht konjugierten mRNA-Fragment gelangt.

16. Verfahren nach Anspruch 12, bei dem das Kopplungsmolekül und das zweite Molekül aus der Gruppe:
a) der biologischen Moleküle, die sich unter Bildung eines nicht kovalenten Komplexes aneinander lagern, sowie
b) Biotinhydrazid als Kopplungsmolekül und entweder Streptavidin oder Avidin als zweites Molekül
stammen.

17. Verfahren nach Anspruch 12, bei dem das zweite Molekül an eine Festphase bindet und die Trennphase die Abtrennung der Festphase der mRNAs bzw. mRNA-Fragmente, die diesen Komplex nicht gebildet haben, umfaßt.

18. Verfahren nach Anspruch 17, bei dem die Festphase aus der Gruppe bestehend aus der Innenseite einer Küvette, in der diese mRNAs bzw. mRNA-Fragmente vorliegen, und Bestandteilen, die in diese Küvette eingeführt werden, stammt.

19. Isoliertes Polynucleotid, umfassend ein nach dem Verfahren von Anspruch 1 oder Anspruch 12 erhaltenes mRNA-Kopplungsmolekül-Konjugat.

20. Verfahren zur Herstellung eines cDNA-Erststrangs, umfassend die folgenden Schritte:
a) Oxidation der 2',3'-cis-Diole an den Cap-Strukturen an den 5'-Enden von Polynucleotiden, umfassend die Eukaryonten-mRNAs bzw. Eukaryonten-mRNA-Fragmente, wodurch man zu Dialehyden gelangt;
b) Kopplung dieser Dialdehyde mit funktionellen Aminogruppen an Kopplungsmolekülen, wodurch Konjugate gebildet werden, die diese Kopplungsmoleküle und diese Polynucleotide enthalten;
c) reverse Transkription dieser Konjugate, wodurch Heteroduplices gebildet werden, die in diesen Konjugaten einen cDNA-Erststrang sowie diese mRNAs bzw. mRNA-Fragmente enthalten;
d) Entfernung der mRNAs bzw. mRNA-Fragmente, die keine solchen Heteroduplices gebildet haben;
e) Gewinnung dieser Heteroduplices an einer Festphase, die mit einem zweiten Molekül, das fähig ist, an das Kopplungsmolekül zu binden, versehen ist, sowie
f) Denaturierung der Heteroduplices, wodurch der cDNA-Erststrang gewonnen wird.

21. Verfahren nach Anspruch 20, bei dem bei der Entfernungsmethode mit einem Enzym, das Einzelstrang- bzw. nicht hybridisierte mRNA bzw. mRNA-Fragmente abbaut, jedoch Heteroduplices nicht angreift, verdaut.

22. Verfahren nach Anspruch 21, bei dem dieses Enzym aus der Gruppe T1-RNAse und S1-Nuclease stammt.

23. Verfahren zur Herstellung von cDNA-Doppelsträngen, die 5'-Enden von Polynucleotiden entsprechen, die Eukaryonten-mRNAs oder Eukaryonten-mRNA-Fragmente enthalten, umfassend die folgenden Schritte:
a) Oxidation der 2',3'-cis-Diole an den auf den 5'-Enden der mRNAs bzw. mRNA-Fragmente gelegenen Cap-Strukturen, wodurch man zu Dialdehyden gelangt,
b) Kopplung einer funktionellen Aminogruppe des 3'-Endes eines Oligonucleotids mit den Dialdehyden, wodurch Oligonucleotid-mRNA-Konjugate gebildet werden;
c) reverse Transkription dieses Oligonucleotid-mRNA-Konjugats, wodurch Heteroduplices gebildet werden, die cDNA-Erststränge und die Oligonucleotid-mRNA-Konjugate enthalten;
d) Denaturierung dieser Heteroduplices;
e) Entfernung der Einzelstrang-mRNAs bzw. -mRNA-Fragmente; sowie
f) Durchführung der Zweitstrang-Synthese von cDNAs, die zu den cDNA-Erststrängen komplementär sind, unter Verwendung eines zur gesamten Sequenz des Oligonucleotids bzw. zu einem Teil der Sequenz des Oligonucleotids komplementären Primers.

24. Verfahren nach Anspruch 23, bei dem die erhaltenen cDNAs vollständigen mRNAs entsprechen.

25. Verfahren zur Gewinnung eines Proteins, das fähig ist, an Eukaryonten-mRNA zu binden, umfassend die folgenden Schritte:
a) Kopplung einer funktionellen Aminogruppe eines Kopplungsmoleküls mit der am 5'-Ende eines Polynucleotids, das eine mRNA oder ein mRNA-Fragment umfaßt, gelegenen Cap-Struktur, wodurch ein Kopplungsmolekül-mRNA-Konjugat gebildet wird;
b) In-Kontakt-bringen des Kopplungsmolekül-mRNA-Konjugats mit einem Protein, das fähig ist, die mRNA bzw. das mRNA-Fragment zu erkennen, wodurch ein Komplex aus dem Protein und dem Kopplungsmolekül-mRNA-Konjugat gebildet wird;
c) Isolation dieses Komplexes;
d) Dissoziation des Proteins von dem Kopplungsmolekül-mRNA-Komplex; sowie
e) Gewinnung des Proteins.

26. Kit zur Gewinnung eines Polynucleotids, enthaltend die 5'-Region einer mRNA oder eines mRNA-Fragments ausgehend von einer Eukaryonten-mRNA-Population, umfassend:
a) ein Modifikationsreagens zur Modifikation der Base am 3'-Ende der mRNA bzw. des mRNA-Fragments, so daß diese Base in 2'- und 3'-Stellung keine OH-Gruppe mehr aufweist;
b) ein Oxidationsmittel zur Oxidation eines 2',3'-cis-Diols auf den an den 5'-Enden der mRNAs bzw. mRNA-Fragmente gelegenen Cap-Strukturen, wodurch man zu Dialdehyden gelangt;
c) ein Kopplungsmolekül mit einer funktionellen Aminogruppe zur Bildung eines Konjugats, umfassend das Kopplungsmolekül und das Polynucleotid;
d) ein Kopplungsreagens zur Kopplung des Dialdehyds mit der funktionellen Aminogruppe.

27. Kit nach Anspruch 26, bei dem das Oxidationsmittel Natriumperiodat ist.

28. Kit nach Anspruch 26, bei dem das Kopplungsreagens einen sauren Puffer mit einem pH zwischen 4 und 6 sowie ein Borhydrid enthält.

29. Kit nach Anspruch 26, bei dem das Kopplungsmolekül ein Molekül aus der Gruppe:
a) ein biologisches Molekül;
b) ein lösliches Molekül;
c) Biotinhydrazid; sowie
d) ein Oligonucleotid mit einer funktionellen Aminogruppe am 3'-Ende
umfaßt.

30. Kit nach Anspruch 26, bei dem das Modifikationsreagens ein Reagens zur Durchführung der Kopplung des 3'-Endes der mRNA bzw. des mRNA-Fragments mit dem 5'-Ende eines Oligonucleotids oder eines Nucleotids umfaßt, wobei das Oligonucleotid bzw. Nucleotid an seinem 3'-Ende kein 2',3'-cis-Diol aufweist.

31. Kit nach Anspruch 30, bei dem das Modifikationsreagens ein pNp-3',5'-Diphosphatnucleotid und ein Ligationsenzym umfaßt.

32. Kit nach Anspruch 31, bei dem das Ligationsenzym aus T4-RNA-Ligase besteht.

33. Kit nach Anspruch 26, das weiterhin Reagentien zur Durchführung einer reversen Transkription umfaßt.

34. Kit zur Gewinnung eines Polynucleotids mit der 5'-Region einer mRNA bzw. eines mRNA-Fragments ausgehend von einer Eukaryonten-mRNA-Population, umfassend:
a) ein Oxidationsmittel zur Oxidation des 2',3'-cis-Diols auf den an den 5'-Enden der mRNAs bzw. mRNA-Fragmente gelegenen Cap-Strukturen, wodurch man zu Dialdehyden gelangt;
b) ein Kopplungsmolekül mit einer funktionellen Aminogruppe zur Bildung eines Konjugats, umfassend das Kopplungsmolekül und das Polynucleotid;
c) ein Kopplungsreagens zur Kopplung des Dialdehyds mit der funktionellen Aminogruppe; sowie
d) ein zweites Molekül, das kovalent oder nichtkovalent an das Kopplungsmolekül bindet und so mit dem Kopplungsmolekül einen Komplex bildet, wobei das zweite Molekül in Lösung vorliegt oder an eine Festphase gebunden ist.

35. Kit nach Anspruch 34, umfassend weiterhin ein Modifikationsreagens zur Modifikation der Base am 3'-Ende der mRNA bzw. des mRNA-Fragments, so daß diese Base in 2'- und 3'-Stellung keine OH-Gruppe mehr aufweist.

36. Kit aus Reagenzien zur Herstellung von Doppelstrang-cDNAs, die den 5'-Enden von Polynucleotiden, umfassend Eukaryonten-mRNAs bzw. Eukaryonten-mRNA-Fragmente, entsprechen, umfassend:
a) ein Oxidationsmittel zur Oxidation des 2',3'-cis-Diol auf einer an den 5'-Enden der mRNAs gelegenen Cap-Struktur, wodurch man zu einem Dialdehyd gelangt;
b) ein Oligonucleotid mit einer funktionellen Aminogruppe an seinem 3'-Ende;
c) ein Kopplungsreagens zur kovalenten Kopplung des Dialdehyds mit dem Oligonucleotid;
d) einen Primer und ein Enzym zur Durchführung einer reversen Transkription dieser mit dem Oligonucleotid gekoppelten mRNAs bzw. mRNA-Fragmente, wodurch man zu Heteroduplices gelangt, die einen cDNA-Erststrang und diese mit dem Oligonucleotid gekoppelte mRNA bzw. gekoppelten mRNA-Fragmente erhält;
e) Reagens zur Denaturierung der Heteroduplices und zur Entfernung der mRNAs bzw. mRNA-Fragmente, so daß man nur die cDNA-Erststränge gewinnt;
f) einen Primer und ein Enzym, mit dem ein cDNA-Zweitstrang synthetisiert werden kann.
